(19) **Europäisches Patentamt / European Patent Office / Office européen des brevets**

(11) **EP 2 518 575 B1**

(12) **EUROPEAN PATENT SPECIFICATION**

(45) Date of publication and mention
of the grant of the patent:
**20.01.2016 Bulletin 2016/03**

(51) Int Cl.:
*G05B 19/4062* $^{(2006.01)}$    *A61M 39/28* $^{(2006.01)}$

(21) Application number: **12165969.2**

(22) Date of filing: **27.04.2012**

(54) **Tube detection for solenoid actuated pinch valves**

Rohrleitungserkennung für Magnetklemmventile

Détection de tube pour valves électro-mécaniques à pincement

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.04.2011 US 201161480077 P**

(43) Date of publication of application:
**31.10.2012 Bulletin 2012/44**

(73) Proprietor: **Acro Associates, Inc.**
**Concord, CA 94520 (US)**

(72) Inventors:
• **Weatherbee, Jamil, J.**
**Pleasant Hills, CA California 94523 (US)**
• **Cousins, Ryan L.**
**Walnut Creek, CA California 94596 (US)**

(74) Representative: **Finnegan Europe LLP**
**16 Old Bailey**
**London EC4M 7EG (GB)**

(56) References cited:
**WO-A1-03/070314        US-A- 5 977 732**
**US-A1- 2010 188 667**

• **HYE-JIN LEE ET AL: "Practical Pinch Detection Algorithm for Low-Cost Anti-Pinch Window Control System", INDUSTRIAL TECHNOLOGY, 2005. ICIT 2005. IEEE INTERNATIONAL CONFERENCE ON HONG KONG 14-17 DEC. 2005, PISCATAWAY, NJ, USA,IEEE, 14 December 2005 (2005-12-14), pages 995-1000, XP010897996, DOI: 10.1109/ICIT.2005.1600781 ISBN: 978-0-7803-9484-1**

EP 2 518 575 B1

**Description**

*Technical Field*

The present disclosure relates generally to the field of fluid control. Fluid control may include, for example, the manner of processing, distributing, or storing of fluids to serve needs in a variety of applications. These include medical, pharmaceutical, biotechnology, laboratory, chemical processing, manufacturing, food and beverage, and industrial applications. More particularly, embodiments of the present disclosure relate to solenoid actuators, such as solenoid pinch valves, that may be utilized within the field of fluid control. Some embodiments relate to techniques and systems for detecting whether or not tubing is properly inserted within solenoid actuators and for controlling solenoid actuators.

*Background*

In fluid control applications, platforms, and systems, such as, for example, those described in U.S. Patent Application Publication Nos. 2010/020074 A1 and 2010/0188667 A1, solenoid actuator valves may be used to pinch tubing to stop (or in some cases reduce) the flow of a fluid (such as liquid or gas) through a tube. Such a valve may be considered part of a fluid control system and may be operated via the controller of such a system. In turn, this controller runs controller software to operate the system. Further, as disclosed in U.S. Patent Application Publication Nos. 2010/020074 A1 and 2010/0188667 A1, and as shown in Figs. 1 and 2, a solenoid pinch valve may be coupled to a position sensor, such as an optical aperture sensor, and an EEPROM or other memory device to form an intelligent fluid control component.

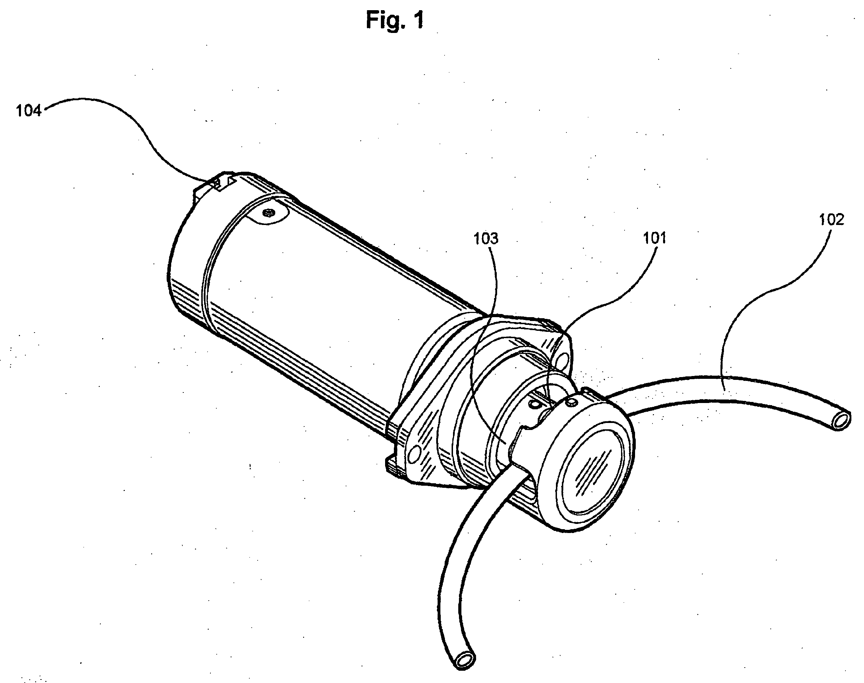

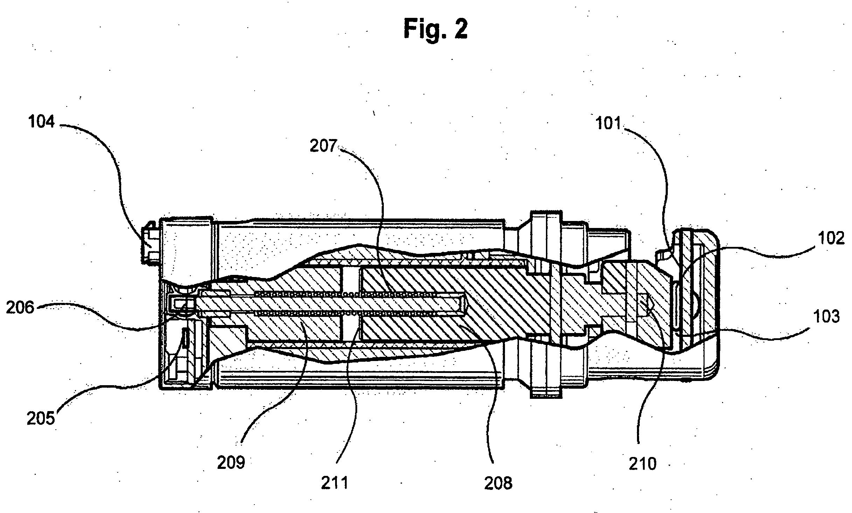

Solenoid pinch valves pinch tubing by compressing flexible tubing between the armature of the solenoid and the armature's contacting surface. For example, as shown in Figs. 1 and 2, a typical solenoid pinch valve has tube slot **103** between the armature **208** (internal portion) / **210** (external portion)and the contacting surface **101** in which flexible tubing **102** may be inserted. Figures 1 and 2 illustrate an example of flexible tubing **102** positioned within tube slot **103** of a solenoid pinch valve. In Fig. 2, a cross-section of flexible tubing **102** is depicted as compressed by armature **208/210.** Additionally, as shown in Fig. 2, it may be noted that, in some embodiments, a solenoid pinch valve at rest (i.e., where no current or where negligible current is transmitted through the solenoid) will close (or will remain closed) due to the force of internal spring **207** on armature **208/210.** As Figs. 1 and 2 depict an intelligent fluid component, optical aperture sensor **206** and EEPROM **205** are also shown. Connector **104** permits communication with the system controller, other control or data collection devices, or respective intermediaries. Finally, fixed pole **209** is a typical element of a solenoid actuator.

Proper compression of tubing may only reliably be effectuated when the tubing is properly positioned between the armature **208/210** and the contacting surface **101.** That is, when flexible tubing **102** is not properly positioned in the tube slot **103,** fluid in the tubing may flow unimpeded, regardless of the state (e.g., open or closed) of the solenoid pinch valve. Moreover, in such a circumstance, the controller of a fluid control system may continue to operate under the incorrect assumption that flow through tube **102** is impeded when the solenoid pinch valve is closed. Here, the system's control of fluid is compromised. Until the tubing is properly repositioned in the tube slot **103,** the fluid control system may malfunction.

As an example, in the case where a fluid control system is part of a medical device, the device may cease to operate properly when tubing is dislodged from its slot. And without information indicating that the tubing has been dislodged, the device cannot effectuate an emergency alert. As a result, a medical provider (or patient) may improperly assume that a patient is still receiving the desired treatment from the medical device, perhaps to the detriment of the patient's heath.

Methods and systems for tube detection may continuously or periodically check to make sure that tubing is properly positioned within a solenoid pinch valve. Such tube detection technology may notify the controller of a fluid control system when a tube is out of place. In turn, the controller may, for example, sound an alarm, provide a visual alert, otherwise notify a user of the malfunction, send electronic signals to notify other systems (or users) of the malfunction, or correct for the error by modifying operation of other fluid control components in a fluid control system.

Some commercially available tube detection methods include direct physical detection of tubing using an optical sensor, a pressure sensor, or a position sensor. The optical sensor method determines the presence of tubing by measuring the passage of light across the slot. A properly positioned tube will block a portion of this light, whereas an improperly positioned (or missing) tube will allow light to pass through the slot unimpeded or otherwise alter the passage of light. This method has the potential drawback of requiring an additional sensor, which in turn must be connected to the controller, further increasing the cost of a fluid control system. Additionally, this tube detection method may malfunction if the light path is completely or partially blocked by, for example, the presence of dirt on the optical sensor.

Pressure sensors may be mounted in the tubing slot and pressure may be measured whenever the valve is closed. In this method, the presence of tubing is detected when the measured pressure is greater than a particular threshold pressure. This method has the potential drawback of requiring an additional sensor, which in turn must be connected to the controller, further increasing the cost of a fluid control system. Additionally, this method may require a different threshold pressure value to be used depending on the type of tubing used. Moreover, as tubing ages or wears, it may become softer and thus, its corresponding threshold pressure value may require adjustment. The requirement of updating the controller software to compensate for tubing type and/or wear represents an additional potential drawback to this method.

Finally, tube presence may be detected by measuring the position of the solenoid pinch valve armature when the valve is closed. Generally, even a compressed tube will prevent a solenoid pinch valve from completely closing. That is, the position of the armature when the valve is closed will be slightly displaced when a tube is present. Although this method may not always require an additional sensor, this method still has its drawbacks. Similarly to the pressure sensing method, this method may require a different threshold position displacement value to be used depending on the type of tubing used. Moreover, as tubing ages and wears, it may become more malleable, requiring adjustment of its corresponding threshold position displacement value. The requirement of updating the controller software to compensate for tubing type and/or age represents a potential drawback to this method.

WO 03/070314 discloses a method of monitoring a flow cut-off member for an extracorporeal fluid circuit and a system for arresting the flow of blood that implements this monitoring method The system comprises a flowcut-off member active on a radially deformable length of tube forming part of the circuit, and a unit capable of causing a movement of a movable portion of the cut-off member towards a closed condition, detecting when the said movable portion passes through a first predetermined position, determining the value assumed by a dynamic parameter associated with the motion of the said movable portion at the first position, and checking that this value is within a predetermined criterion of acceptability. US 5977732 discloses an apparatus and method for determining whether a foreign object or the like is caught in an open-and-closure mechanism such as a power window of an automotive vehicle. An external disturbance torque T imposed on a the open- and-closure mechanism motor as its load is calculated on the basis of at least two of three parameters of a terminal voltage Em across the motor, a current flowing through the motor Im, and a motor angular velocity omega. Furthermore, the calculated external disturbance torque is differentiated to derive a change rate at which the external disturbance torque T is changed with time as dT/dt (T'). Since dT/dt (T')=dTs'/dt (Ts')+dTd'/dt (Td') and dTd/dt (Td')=O (Td: a dynamic friction resistance force and dTdldt: the change rate of the dynamic friction resistance force per time), dT/dt=dTs'/dt (Ts: an external disturbance force imposed on the motor by the caught foreign object and dTs/dt (Ts'): the differentiation value of the external disturbance force). When the change rate (dT/dt or T') of the external disturbance torque Tis compared with a predetermined threshold value (Kw in a second embodiment), an accurate determination of whether the foreign object or the like is caught in the open-and-closure mechanism can be made since an aged deterioration and/or a temperature variation in the dynamic friction resistance Td are almost not included in the change rate (dT/dt, T') at which the external disturbance torque Tis changed with time. HYE-JIN LEE et al: "Practical Pinch Detection Algorithm for Low-Cost Anti-Pinch Window Control System", INDUSTRIAL TECHNOLOGY, 2005. ICIT 2005. IEEE INTERNATIONAL CONFERENCE ON HONG KONG 14-17 DEC. 2005, PISCATAWAY, NJ, USA,IEEE, 14 December 2005, pages 995-1000 discloses a pinch torque estimator based on the H∞ filter for low-cost anti-pinch window control systems. To obtain the acceptable angular velocity measurements from Hall-effect sensor outputs, the angular velocity calculation algorithm is proposed with the measurement noise reduction logics. The proposed pinch detection algorithm makes use of the torque rate information. To do this, the torque rate is augmented to the system model and the torque rate estimator is derived by applying the steady-state H∞ filter recursion to the model.

## SUMMARY OF THE DISCLOSURE

Systems and methods according to certain embodiments of the present disclosure include techniques and systems for detecting tubing in a solenoid actuated valve.

One aspect of the invention provides a method of detecting tubing in a solenoid actuated pinch valve comprising measuring operation of an armature of the pinch valve; the method being characterized by providing calibration data pertaining to the solenoid pinch valve; calculating an amount of work performed by the armature of the solenoid actuator; comparing the amount of work performed to a threshold value; and determining a presence of tubing in the solenoid pinch valve based on a result of the comparison.

Another aspect of the invention provides system for detecting tubing in a solenoid actuated pinch valve, comprising a solenoid actuator with an armature, wherein the solenoid actuator is configured to receive the tubing and to measure operation of the armature; and a data storage device; characterized in that the data storage device contains calibration data pertaining to the solenoid actuator; and wherein the system further comprises a controller configured to calculate an amount of work performed by the armature, compare the amount of work performed by the armature to a threshold value, and determine a presence of tubing in the solenoid pinch valve based on the comparison results.

**BRIEF DESCRIPTION OF THE DRAWINGS**

Figure 1 is a depiction of an intelligent fluid control component comprising a solenoid pinch valve, consistent with an embodiment of the invention.

Figure 2 is a partial cross section of the intelligent fluid control component depicted in Figure 1, further showing an optical aperture sensor and an EEPROM, consistent with an embodiment of the invention.

Figure 3 is a table providing identification information and calibration information pertaining to a solenoid pinch valve, consistent with an embodiment of the invention.

Figure 4 is a table providing calibration information pertaining to a solenoid pinch valve with regard to pull-in current, hold-in current, drop-out current, and their corresponding armature positions, consistent with an embodiment of the invention.

Figure 5 is a table providing calibration information pertaining to a position sensor coupled to a solenoid pinch valve, consistent with an embodiment of the invention.

Figure 6 is a table providing calibration information pertaining to a solenoid pinch valve with regard to calculating null current values and di/dF values, consistent with an embodiment of the invention.

Figure 7 is a graph corresponding to values calculated in Fig. 6, depicting backward and forward di/dF as functions of position for a solenoid pinch valve as the valve closes and opens, respectively, consistent with an embodiment of the invention.

Figure 8 is a graph corresponding to values calculated in Figs. 4 and 6, depicting backward and forward null current as functions of position for a solenoid pinch valve as the valve closes and opens, respectively, consistent with an embodiment of the invention.

Figure 9 is a graph corresponding to values from Fig. 5, depicting position sensor voltage output as a function of position for a solenoid pinch valve, consistent with an embodiment of the invention.

Figure 10 is a graph corresponding to values from Fig. 5, depicting position as a function of position sensor voltage output for a solenoid pinch valve, consistent with an embodiment of the invention.

Figure 11 is a flow chart depicting a method of acquiring calibration data during a forward pass (e.g., associated with the opening of a valve), consistent with an embodiment of the invention.

Figure 12 is a flow chart depicting a method of acquiring calibration data during a backward pass (e.g., associated with the closing of a valve), consistent with an embodiment of the invention.

Figure 13 is a flow chart depicting a method of tube detection, consistent with an embodiment of the invention.

Figure 14 is a graph depicting the relationship between armature position, current through the valve, force exerted by the valve, and work completed by the valve as a function of time for a solenoid pinch valve that is closing, is oriented horizontally, and does not contain tubing, consistent with an embodiment of the invention.

Figure 15 is a graph depicting the relationship between armature position, current through the valve, force exerted by the valve, and work completed by the valve as a function of time for a solenoid pinch valve that is closing, is oriented horizontally, and contains tubing, consistent with an embodiment of the invention.

Figure 16 is a graph depicting the relationship between armature position, current through the valve, force exerted by the valve, and work completed by the valve as a function of time for a solenoid pinch valve that is closing, is oriented vertically, and does not contain tubing, consistent with an embodiment of the invention.

Figure 17 is a graph depicting the relationship between armature position, current through the valve, force exerted by the valve, and work completed by the valve as a function of time for a solenoid pinch valve that is closing, is oriented vertically, and contains tubing, consistent with an embodiment of the invention.

Figure 18 is a graph depicting the relationship between armature position and current through the valve as a function of time for a solenoid pinch valve that is opening, is oriented horizontally, and does not contain tubing, consistent with an embodiment of the invention.

Figure 19 is a graph depicting the relationship between armature position and current through the valve as a function of time for a solenoid pinch valve that is opening, is oriented horizontally, and contains tubing, consistent with an embodiment of the invention.

Figure 20 is a graph depicting the relationship between armature position and current through the valve as a function of time for a solenoid pinch valve that is opening, is oriented vertically, and does not contain tubing, consistent with an embodiment of the invention.

Figure 21 is a graph depicting the relationship between armature position and current through the valve as a function of time for a solenoid pinch valve that is opening, is oriented vertically, and contains tubing, consistent with an embodiment of the invention.

Figure 22 is a table depicting the calculated amount work done by the armature of a solenoid pinch valve model in compressing various types of tubing in multiple trials, consistent with an embodiment of the invention.

Figure 23 is a flow chart depicting a simplified overview of a method of electomechanical damping, consistent with an embodiment of the invention.

Figure 24 is a flow chart depicting a method of determining the likelihood of tube dislodgement in a closed solenoid pinch valve, consistent with an embodiment of the invention.

## DETAILED DESCRIPTION

Reference will now be made in detail to the exemplary embodiments of the disclosure, examples of which are illustrated in the accompanying drawings. Wherever possible, the same reference numbers will be used throughout the drawings to refer to the same or like parts.

This disclosure relates to a tube detection method that electronically measures work done by a solenoid pinch valve armature as the valve closes. Because changes in velocity and the effect of gravity can be assumed to be negligible (or may be otherwise compensated for as discussed herein), the work done by the solenoid armature may generally be understood to represent the work needed to compress tubing, if such tubing is present. By comparing the amount of work done by the armature to a threshold work value, this method can determine the absence or presence of tubing.

When the magnitude of the work done is greater than (or, in some embodiments, equal to) the magnitude of the threshold work value, the presence of tubing in tube slot **103** may be confirmed. And when the magnitude of the work done is less than (or, in other embodiments, equal to) the magnitude of the threshold work value, the tubing may have been dislodged from tube slot **103.** Although this "work method" of tube detection may (similarly to the position sensing method discussed earlier) employ an armature position sensor, a threshold work value may be selected such that the presence of tubing may be detected virtually without regard to a tube's type, age, or wear.

At a given armature position, a solenoid pinch valve's armature does not accelerate (in either direction) when the valve is provided with a particular amount of current. This current may be called the null current, $I_{null}(X)$, and this characteristic is a function of armature position, X. dF/di(X) is a characteristic of a solenoid pinch valve that describes the armature's change in force relative to the change in current through the valve at a particular position, X. Similarly, di/dF(X) is a characteristic of a solenoid pinch valve that describes the change in current through the valve relative to the armature's change in force at a particular position, X. It should be noted that dF/di(X) and di/dF(X) are typically linear approximations that are estimated with respect to $I_{null}(X)$.

In one embodiment, a controller may control a fluid control system by continuously cycling through a higher level controller software function. In one embodiment, each such cycle takes approximately 40 micro-seconds. For example, in each cycle, the controller may measure the actual current flowing through a solenoid pinch valve-$I_{actual}$-and (via a position sensor) the position of the armature-X. This data may be used for tube detection. And in another embodiment, a tube detection method may not begin summing work completed until the armature has reached a steady, constant velocity.

Because the velocity is the same (i.e., 0 in. / sec. (0 mm/sec)) at the beginning and end of the closing of the valve and because the effect of gravity is assumed to be negligible (or otherwise compensated), it may be assumed that any deviation of the measured current from the null current is the result of force being exerted on the armature by a tube (or of noise in the system). Thus, multiplying this deviation in current by the appropriate dF/di value-herein defined as 1/(di/dF)-should yield the incremental amount of force being exerted. Then, by multiplying the incremental amount of force by the incremental change in position, this method can determine the incremental amount of work exerted upon an inserted tube (if any). Finally, the amount of work, determined by summing incremental amounts of work, may be compared to a threshold work value. This threshold work value is less than the amount of work required to close the valve while compressing virtually any tube and greater than the amount of work required to close the valve absent any tubing.

This disclosure also relates to systems and techniques that determine the likelihood of whether flexible tubing within a pinch valve has been dislodged by measuring the velocity of the armature of a solenoid pinch valve. Such systems and techniques may be used to monitor a closed solenoid.

This disclosure also relates to a new electronic damping technique that utilizes null current values and di/dF values to control the velocity of the armature of a solenoid pinch valve.

Additionally, this disclosure relates to calibration techniques that may be used to measure calibration values, such as the correlation between armature position and sensor voltage output, the pull-in current, the hold-in current, and the drop-out current. In turn, these measured calibration values may be used to calculate additional calibration values representing, for example, $I_{null}(X)$ and di/dF(X). Additionally, it may be noted that $I_{null}(X)$ and di/dF(X) may have different values depending on whether a solenoid pinch valve is opening or closing, and the disclosed calibration techniques describes how these respective values may be derived.

*CALIBRATION*

As described in U.S. Patent Application Publication Nos. 2010/020074 A1 and 2010/0188667 A1, a fluid control component such as a solenoid pinch valve may need to be calibrated to perform certain tasks or effectuate certain algorithms. The tube detection methods disclosed herein may require some calibration. Techniques for achieving such calibration are described below. Further as described in U.S. Patent Application Publication Nos. 2010/020074 A1 and 2010/0188667 A1 and depicted in Figs. 1 and 2, where a solenoid pinch valve is part of an intelligent fluid control component, calibration data may be stored in an EEPROM that comprises part of the component. Alternatively, the calibration data may be stored almost anywhere else, including for example, memory associated with the controller, so long as it may be accessed by the controller.

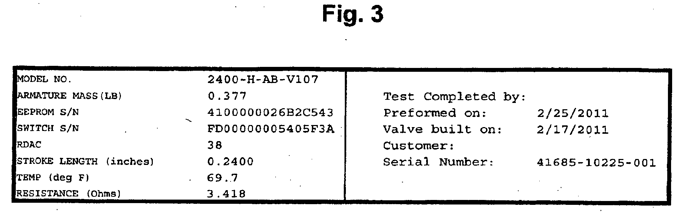

Figs. 3 -10 describe the characteristics of an intelligent fluid control component comprising a solenoid pinch valve and a position sensor, consistent with embodiments of the invention. That is, these figures represent a set of measured, observed, and calculated calibration data for a particular solenoid pinch valve. Specifically, Figs. 3-10 describe the characteristics of the solenoid pinch valve identified in Fig. 3 by Model and EEPROM serial number (used as the valve serial number), and switch serial number (which may be used for serial communication over the I2C bus). It may be noted that "RDAC" stands for Resistive Digital Analog Converter. This is a value that represents a calibration value (optical gain) to achieve the best range for the light sensor within the optical aperture sensor.

The calibration techniques disclosed herein may be effectuated by a custom-created calibration instrument for solenoid pinch valves. This calibration instrument may be comprised of a fixture with mounting clamps, which may hold the solenoid valve in place; slide(s), such as ball slide(s), or other low friction slides that may removably attach to and move with the armature of a solenoid pinch valve; a commercially available stepper motor to move and/or put pressure on the solenoid armature; a force gauge to measure force exerted by (or on) the armature; and a position sensor or indicator to measure the position of the armature relative to the valve. In one embodiment, the stepper motor may exert pressure on the armature via the slide(s). Similarly, the force gauge and position sensor may respectively measure force and position via the slide(s).

In one embodiment, a desktop computer using LabVIEW, or similar software, may both control and acquire data from the above-described calibration instrument.

*POSITION SENSOR CALIBRATION*

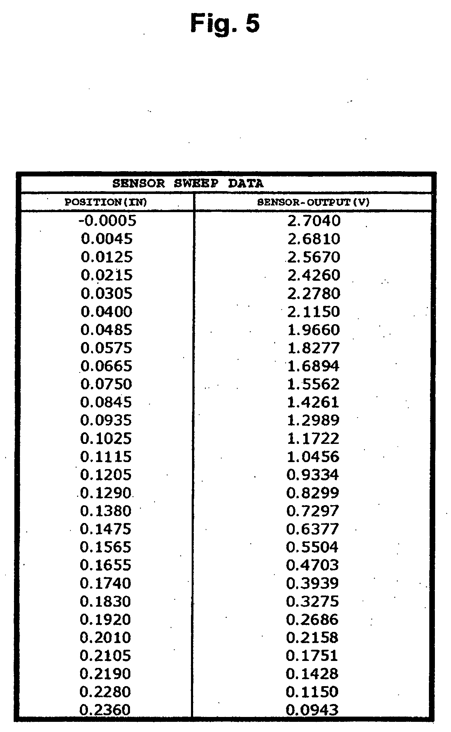

Fig. 5 depicts data from the calibration of an optical aperture sensor (which is described at length in U.S. Patent Application Publication Nos. 2010/020074 A1 and 2010/0188667 A1). The table of Fig. 5 represents measured calibration data for such a sensor attached to a solenoid pinch valve. The left column indicates the relative position of the optical aperture sensor pin (and thus the armature of the pinch valve) and the right column indicates the corresponding output voltage from the optical aperture sensor. When the position is at its minimum value-approximately 0 inches (0 mm)-the valve may be understood to be closed. And when the position is at its maximum value-approximately the stroke length-then valve may be understood to be open. In this embodiment, the pin of an optical aperture sensor is coupled to the armature of the solenoid pinch valve. As such, the relative position of the pin directly correlates to the relative position of the armature of the solenoid pinch valve.

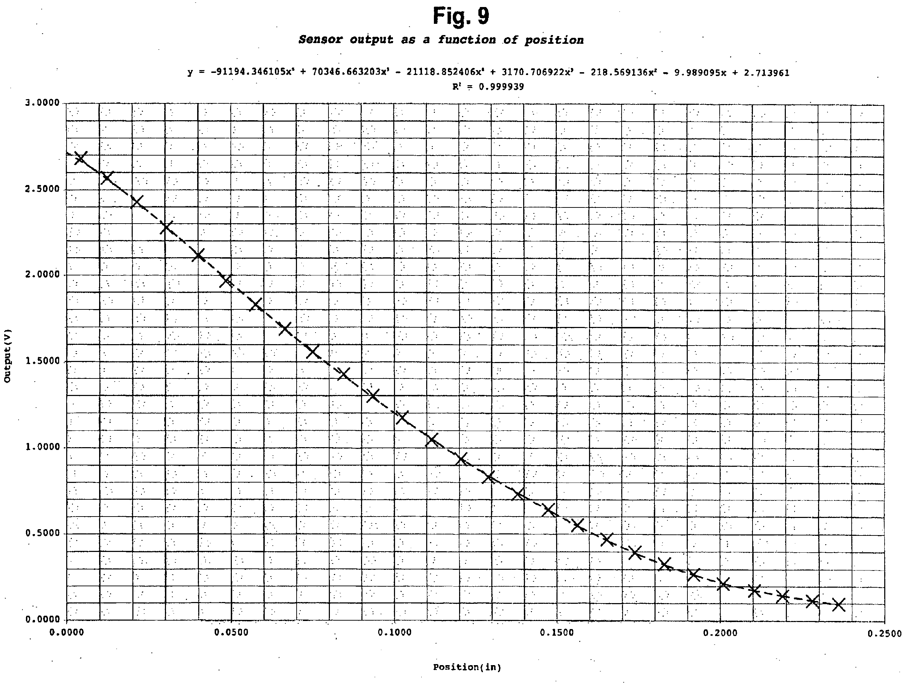

Figure 9 is a graph of position sensor output (in volts) as a function of position. It is a representation of the data depicted in Fig. 5. A best fit curve may be calculated from this data. As an example, the polynomial representing this best fit curve is written above the graph of Fig. 9. Further, the coefficients from the polynomial expressing this best fit curve may be loaded into an EEPROM as calibration data. Alternatively, a table of position and sensor output values may be loaded into an EEPROM as calibration data.

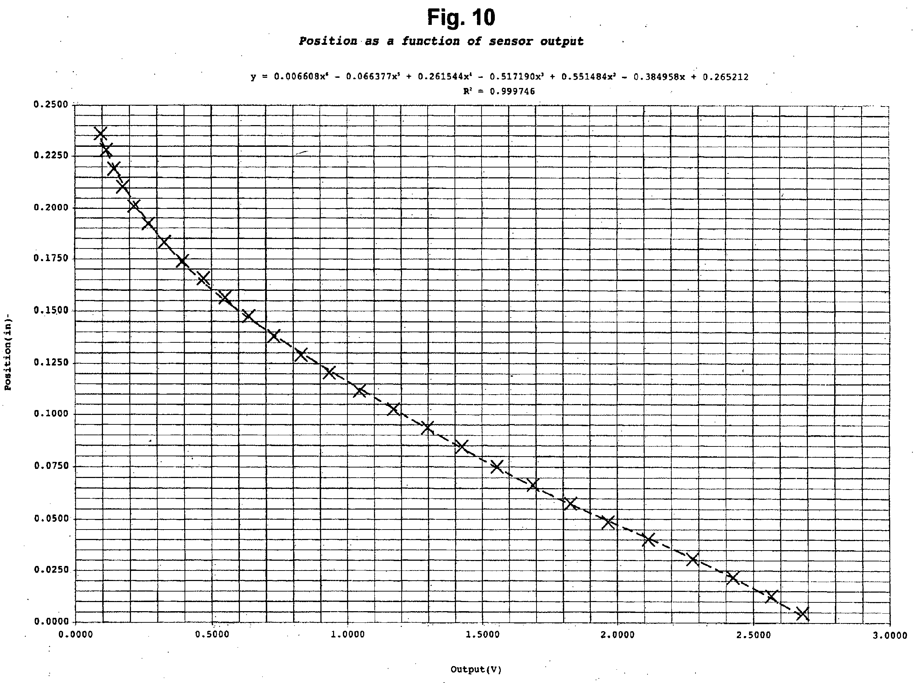

Fig. 10 is a graph of position as a function of position sensor output (in volts). It is a representation of the data depicted in Fig. 5. A best fit curve may be calculated from this data. As an example, the polynomial representing this best fit curve is written above the graph of Fig. 10. Further, the coefficients from the polynomial expressing this best fit curve may be loaded into an EEPROM as calibration data. Alternatively, a table of position and sensor output values may be loaded into an EEPROM as calibration data.

Although position is measured in inches in this embodiment, some position measurements may be less than 0, as shown in Fig. 5. This may result from mechanical noise in the calibration fixture and/or may reflect permissible calibration tolerances. However, due to the relative nature of these position measurements, the position data measurements may be corrected for this negative offset such that a fully closed valve would result in a position measurement of 0 inches (0 mm), and a fully open valve may result in a position measurement very near to the stroke length. For example, a correction offset could be effectuated in controller software. That is, the controller software could add 0.0005 to each value in the position column of Fig. 5; then, the first data point would be equal to 0 inches (0 mm) and the last would be equal to the stroke length, 0.2365 inches (6.007 mm) (compare with 0.2400 in. (6.096 mm) stroke length shown in Fig. 3.) Solenoid valves are typically constructed with elastomer pad 211 positioned between the internal portion of the armature 208 and its fixed pole 209. As shown in Fig. 2, elastomer pad 211 may be attached to armature 208, and travel with it as the valve opens and closes. Alternatively, elastomer pad 211 may be attached to fixed pole 209. It may be noted that the stroke length is typically calculated with the elastomer pad virtually fully compressed. By contrast, the above described sensor calibration data (shown in Fig. 5) is typically taken without the elastomer pad being fully compressed. This results in a small difference in the measured position value.

Alternatively, the position data could be corrected prior to loading a representation of the calibration data into an EEPROM coupled to the sensor. As yet another alternative, such calibration data may be used without any offset correction.

*PULL-IN CURRENT, HOLD-IN CURRENT, AND DROP-OUT CURRENT CALIBRATION*

A solenoid pinch valve has the inherent properties of a pull-in current-$I_{pull}$, a hold-in current-$I_{hold}$, and a drop out current-$I_{drop}$.

The pull-in current is the minimum current required to begin opening a solenoid pinch valve.

The hold-in current is the minimum current required to safely keep an opened valve fully open. That is, when $I_{hold}$ is supplied to a solenoid pinch valve, not only should the valve remain open, but elastomer pad 211 between the armature 208/210 and fixed pole 209 in the valve is virtually fully compressed. Generally speaking, the fixed pole 209 serves as the "stop" in the valve, and enables a solenoid pinch valve to remain open with a relatively low current. Elastomer pad 211 serves to reduce mechanical impact and wear on armature 208/210 and fixed pole 209 from impacts generated when the solenoid actuator is opened. Importantly, elastomer pad 211 also prevents the solenoid valve from sticking in the open position due to residual magnetic or other physical effects, thereby ensuring that spring 207 (a fail-safe that keeps the valve closed as a default position) will work consistently.

Another inherent property of a solenoid punch valve is the drop-out current-$I_{drop}$. The drop-out current is the minimum current required to keep an opened valve open. That is, when $I_{drop}$ is supplied to a solenoid pinch valve, the valve should remain open, but elastomer pad 211 between the armature and the fixed pole is only slightly compressed. When less current than $I_{drop}$ is supplied to the valve, the armature "drops out" of its static, open position and begins to close. Further, a current of $I_{drop}$ does not reliably keep the valve open. That is, when $I_{drop}$ is provided to a solenoid pinch valve, a slight external physical impact to the valve may cause it to close.

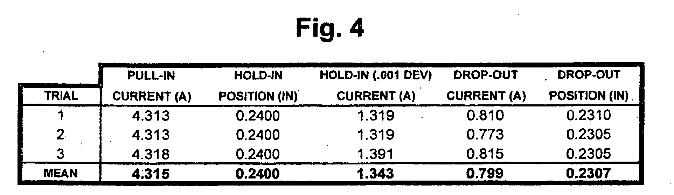

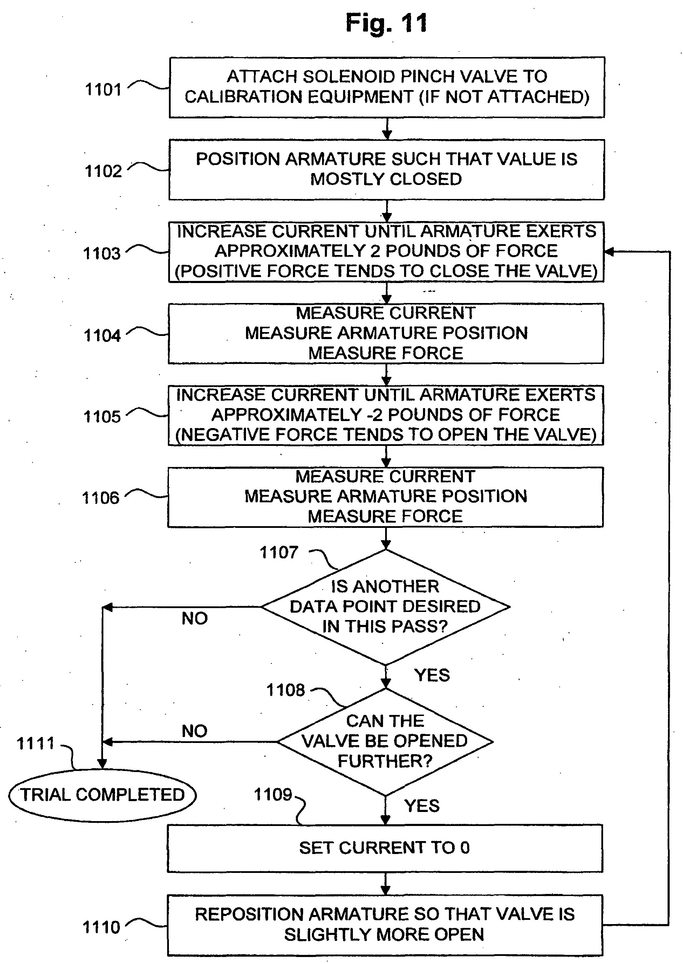

Fig. 4 reflects calibration data pertaining to $I_{pull}$, $I_{hold}$, and $I_{drop}$. (U.S. Patent Application Publication Nos. 2010/020074 A1 and 2010/0188667 A1, also describe a method of determining these values. *See* Fig. 11 of those publications, respectively.)

To measure $I_{pull}$, the valve is permitted to close and the position is measured. This "pull-in position" is equal to $X_{closed}$. (Further, where $X_{closed}$ is not measured at 0, it may be assumed to be equal to 0, wherein all other position values may be similarly offset.) Then, the current is slowly increased until the armature position suddenly shifts toward the open position. The last measured current prior to the sudden shift may be considered $I_{pull}$.

To measure $I_{hold}$, the valve is opened (by means of sufficient current-for example, $I_{pull}$-flowing through the valve) and the fully open position is measured. This may be considered the "hold-in position"-$X_{hold}$. At the hold-in position, the elastomer pad may be.considered to be virtually fully compressed. Then, the current is decreased until the armature position changes slightly-but still remains closed. The last measured current may be considered $I_{hold}$. (Although U.S. Patent Application Publication Nos. 2010/020074 A1 and 2010/0188667 A1 describe a method of determining $I_{hold}$, that method does not account for two separate calibration values of $I_{hold}$ and $I_{drop}$. Rather, the value of $I_{hold}$ as disclosed in U.S. Patent Application Publication Nos. 2010/020074 A1 and 2010/0188667 A1 is generally an intermediate value between the two separate calibration values of $I_{hold}$ and $I_{drop}$ disclosed herein.)

To measure $I_{drop}$, the valve may be placed in hold-position (by way of sufficient current flowing through the valve). The current is incrementally decreased and the position of the armature is measured. If the valve remains open, the current is incrementally decreased again, and the position of the armature is measured again. This cycle continues until the armature "drops out," i.e., the valve suddenly shifts toward the closed position. The last measured current prior to closure may be considered $I_{drop}$ and the last measured armature position may be considered the "drop-out position" -$X_{drop}$.

In one embodiment, $I_{hold}$, $I_{pull}$, $I_{drop}$, and their corresponding positions may be measured for three or more trials. The measurements from these trials may be averaged to yield more accurate values for $I_{hold}$, $I_{pull}$, $I_{drop}$, and their corresponding positions. However, one or two trials may also prove sufficient to provide accurate values for $I_{hold}$, $I_{pull}$, $I_{drop}$, and their corresponding positions.

*NULL CURRENT CALIBRATION*

As described in U.S. Patent Application Publication Nos. 2010/020074 A1 and 2010/0188667 A1, for a given armature position, the armature does not accelerate (in either direction) when the solenoid actuator is provided with a particular amount of current. This amount of current may be described as the non-acceleration current-$I_{non-acceleration}(X)$-or, herein, as the null current-$I_{null}(X)$. The null current may be described as a function of armature position-X. That is, when $I_{null}(X)$ flows through the solenoid valve, the armature (which is at position X) does not tend to accelerate because when this particular amount of current is provided, the net force acting upon the armature is virtually negligible. It should be noted that the system is unstable, so without applying a position feedback control algorithm (including adjusting the current accordingly), the armature will not remain in a static position.

U.S. Patent Application Publication Nos. 2010/020074 A1 and 2010/0188667 A1 disclose determining $I_{null}(X)$ by measuring the pull-in current and the hold-in current for a particular valve, and then estimating an $I_{null}(X)$ curve from these values. However, as described below, there are other ways to determine $I_{null}(X)$.

Due to, *inter alia,* friction between the physical elements of a solenoid actuator, the null current value at a particular position may differ depending on the direction in which the armature is moving. The armature may be considered to be moving forward when the valve is opening (i.e., the armature is moving against the force of the spring 207). And the armature may be considered to be moving backward when the valve is closing (i.e., the armature is moving with the force of the spring.) That is, in this calibration embodiment, $I_{null}(X)$ may have a forward value and a backward value for each value of X. $I_{F-null}(X)$ may be understood to represent the forward null current value as a function of armature position. $I_{B-null}(X)$ may be understood to represent the backward null current value as a function of armature position.

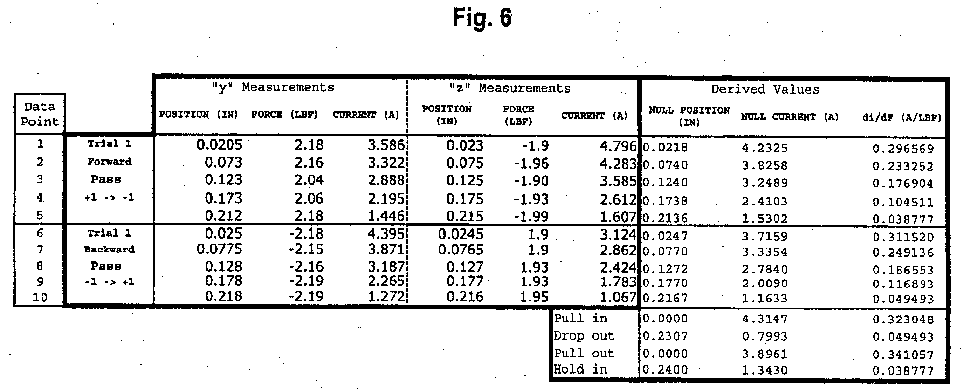

Fig. 6 depicts calibration measurements taken with respect to a particular solenoid pinch valve, which may be used to determine $I_{F-null}(X)$ and $I_{B-null}(X)$, as well as other calibration values. The data in Fig. 6 includes data from a single trial, which includes a "forward pass" and a "backward pass."

The columns titled "POSITION (IN)" describe the position of the armature in inches. A value of near zero indicates that the valve is closed. Because some mechanical clearance from the armature's hard stops is required by the calibration equipment to measure force, the valve may not fully close in this calibration embodiment. The columns titled "FORCE (LBF)" describe the force exerted by the armature in pounds (0.454 kgf). A positive value indicates that the valve is closing. Finally, the columns titled "CURRENT (A)" describe the amount of current supplied to the solenoid actuator in Amperes.

The data represented by Fig. 6 may be obtained as follows. A flow chart of an embodiment of the forward pass data point measurement method is shown in Fig. 11. Prior to beginning the calibration routine, the calibration equipment should be properly attached to the solenoid valve, as in step 1101, and the valve may be positioned so that it is mostly closed, as in step 1102.

Then, the "forward pass" portion of trial 1 may begin. With reference to the "y" measurements of the data point 1, the current flowing through the solenoid is increased until the armature exerts approximately 2 lb. of force (0.908 kgf) (towards closing the valve) on the calibration equipment, as in step 1103 of Fig 11. With reference to the example of Fig. 6, the current was increased until 2.18 lb. (0.990 kgf) was exerted. This calibration value may be referred to as F[1y] and is measured and recorded, as in step 1104. Also, as in step 1104, the position of the armature-X[1y] = 0.0205 in..5207 mm) (0-and the amount of current-I[1y] =3.586 A-are recorded.

Then, with reference to the "z" measurements of data point 1, the current flowing through the solenoid is increased until the armature exerts approximately -2 lb. of force (-0.908 kgf) (i.e., about 2 lb (0.908 kgf) toward opening the valve) on the calibration equipment, as in step 1105. Here, the current was increased until -1.9 lb. (-0.863 kgf) was exerted. This calibration value may be referred to as F[1z] and is measured and recorded, as in step 1106. Also, as in step 1106, the position of the armature-X[1z] = 0.023 in. (0.134 mm)-and the amount of current-I[1z] = 4.796 A-are recorded.

As long as another data point is desired, as in step 1107, and as long as the valve can be opened further, as in step 1108, the trial continues. If either another data point is not desired or the valve cannot be open further, then the trial is completed as in step 1111. Where the trial continues, the current flowing through the valve may be set to 0, as in step 1109. With reference to data point 2, the position of the armature is moved slightly (i.e., the solenoid pinch valve is opened more), as in step 1110. Then, the process described above with respect to data point 1 is repeated, as in steps 1103-1108. That is, both "y" and "z" measurements are taken in the same manner. As shown in the example of Fig. 6, This "forward pass" portion of the first trial continues through data point 5. And at the fifth data point, the solenoid pinch valve is mostly open. Because each data point is taken at a different armature position, the five data points can be understood to describe representative characteristics of the solenoid pinch valve when it is opening. In other embodiments of this calibration method, 6, 7, or more data points may ultimately provide more accurate results.

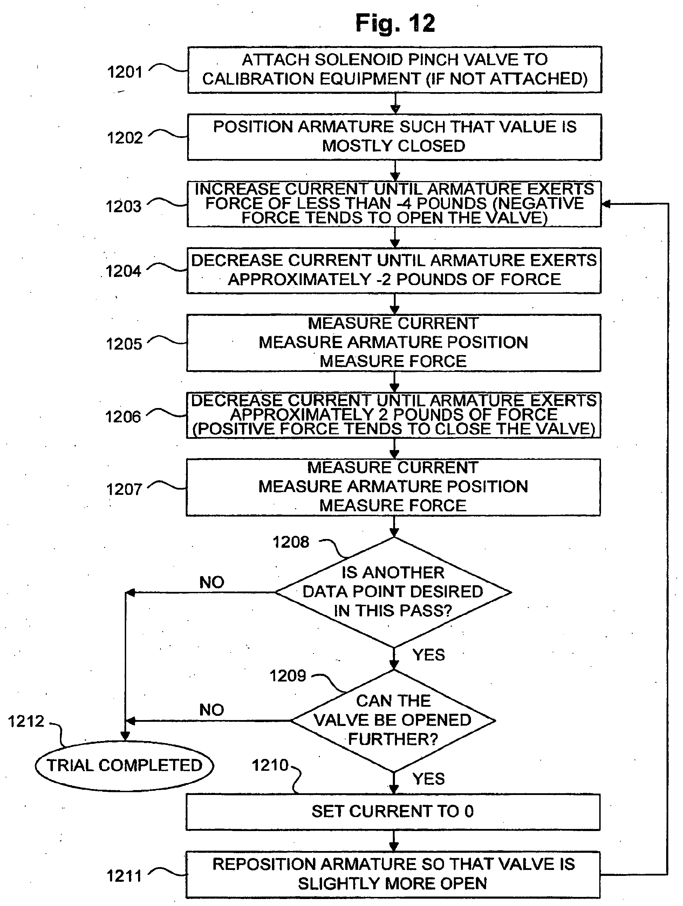

A flow chart of an embodiment of the backward pass data point measurement method is shown in Fig. 12. Prior to beginning the calibration routine, the calibration equipment should be properly attached to the solenoid valve, as in step 1201. Then, the "backward pass" portion of trial 1 may begin. With reference to the "y" measurements of data point 6, the valve is again positioned so that it is nearly closed, as in step 1202. The current flowing through the solenoid pinch valve is increased such that the armature exerts significant force toward opening the solenoid valve, as in step 1203. Then, the current flowing through the solenoid is decreased until the armature exerts approximately -2 lb. of force (-0.908 kgf) (i.e., about 2 lb (0.908 kgf) toward opening the valve) on the calibration equipment, as in step 1204. With reference to the example of Fig. 6, the current was decreased until -2.18 lb. (-0.990 kgf) was exerted. This calibration value may be referred to as F[6y] and is measured and recorded, as in step 1205. Also, as in step 1205, the position of the armature-X[6y] = 0.025 in. (0.635 mm)-and the amount of current-I[6y] =4.395 A-are recorded.

Then, with reference to the "z" measurements of data point 6, the current flowing through the solenoid is further decreased until the armature exerts approximately 2 lb. of force (0.908 kgf) (toward closing the valve) on the calibration equipment, as in step 1206. Here, the current was decreased until 1.9 lb. (0.863 kgf) was exerted. This calibration value may be referred to as F[6z] and is measured and recorded, as in step 1207. Also, as in step 1207, the position of the armature-X[6z] = 0.0245 in. (0.622 mm)-and the amount of current-I[6z] = 3.124 A-are recorded.

As long as another data point is desired, as in step 1208, and as long as the valve can be opened further, as in step 1209, the trial continues. If either another data point is not desired or the valve cannot be open further, then the trial is completed as in step 1212. Where the trial continues, the current flowing through the valve may be set to 0, as in step 1210. With reference to data point 7, the position of the armature is moved slightly (i.e., the solenoid pinch valve is opened more), as in step 1211. Then, the process described above with respect to data point 6 is repeated, as in steps 1203-1209. That is, both "y" and "z" measurements are taken in the same manner. As shown in the example of Fig. 6, this "backward pass" portion of the first trial continues through data point 10. And at the tenth data point, the solenoid pinch valve is mostly open. Because each data point is taken at a different armature position, the five data points can be understood to describe representative characteristics of the solenoid pinch valve when it is closing. In other embodiments of this calibration method, 6, 7, or more data points may ultimately provide more accurate results.

In other embodiments, two, three, or more additional calibration trials may conducted, each including a forward pass and a backward pass. Such additional trials may result in more accurate measured and calculated calibration values. It may be noted that the order of the trials should not affect the resulting calibration data.

For each data point, a "null position" is calculated. Although, the values of X[#y] and X[#z] should theoretically be identical for any given data point #, due to flex in the calibration system and other factors, these y and z measurements may not be identical. For example, strain on the calibration system due to the exertion of force in divergent directions (i.e., to respectively determine y and z values) may cause the calibration fixture to flex or shift. Thus a null position value-$X_{null}$[#]-is calculated to represent the armature position at data point #. This $X_{null}$[#] may be determined by a two-dimensional geometric interpolation according the following formula:

$$X_{null}[\#]= X[\#y] + \frac{(-F[\#y]) * (X[\#z]- X[\#y])}{(F[\#z] - F[\#y])}$$

For example, for data point 1, $X_{null}$[1] may be calculated as follows:

$$X_{null}[1]= X[1y] + \frac{(-F[1y]) * (X[1z]- X[1y])}{(F[1z] - F[1y])}$$

For each data point, a null current is calculated that corresponds with the null position. This may be noted as $I_{null}$[#]. Further, this null current may be understood to be either $I_{F-null}(X_{null}[\#])$ or $I_{B-null}(X_{null}[\#])$, depending on whether the data point is from a "forward pass" or "backward pass," respectively. For example, where # is a "forward pass" data point, $I_{null}[\#] = I_{F-null}(X_{null}[\#])$. Similarly, where # is a "backward pass" data point, $I_{null}[\#] = I_{B-null}(X_{null}[\#])$. That is, both $I_{F-null}$ and $I_{B-null}$, may be considered to be functions of X, where $X=X_{null}$.

For each data point, a value of $I_{null}$[#] may be determined by a two-dimensional geometric interpolation according the following formula:

$$I_{null}[\#]= I[\#y] + \frac{(-F[\#y]) * (I[\#z]- I[\#y])}{(F[\#z] - F[\#y])}$$

For example, for data point 1, $I_{null}$[1] may be calculated as follows:

$$I_{null}[1]= I[1y] + \frac{(-F[1y]) * (I[1z]- I[1y])}{(F[1z] - F[1y])}$$

And because $I_{null}$[1] was calculated from a "forward pass," it may be understood to be $I_{F-null}$.

Similarly, for data point 6, $I_{null}[6]$ may be calculated as follows:

$$I_{null}[6] = I[6y] + \frac{(-F[6y]) * (I[6z] - I[6y])}{(F[6z] - F[6y])}$$

And because $I_{null}[6]$ was calculated from a "backward pass," it may be understood to be $I_{B-null}$.

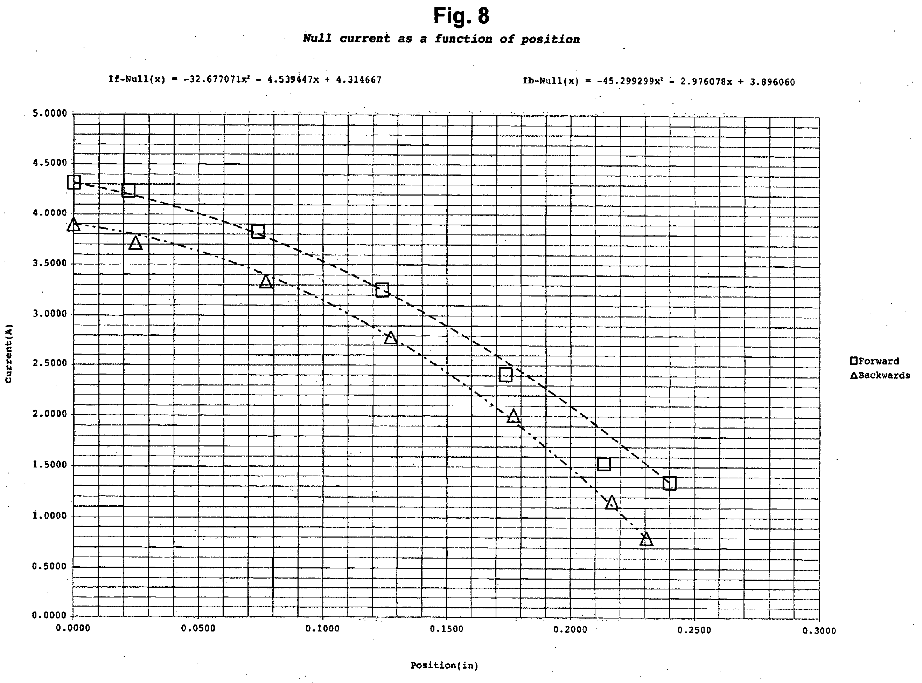

As depicted in Fig. 8, once null current is calculated for each data point, those values-for the forward pass and the backward pass, respectively-may be graphed and/or best-fit curves created. Further, as depicted in Fig. 8, the pull-in current (see above and Fig. 4) may be included as an additional data point for the forward pass. A position of $X_{closed}$ (typically $X_{closed}$ = 0 in. (0 mm)) corresponds with the pull-in current. That is, $I_{F-null}(X_{closed}) = I_{pull}$.

The hold-in current (see above and Fig. 4) may be included as another data point for the forward pass. The hold-in position corresponds with $I_{hold}$. That is, $I_{F-null}(X_{hold}.) = I_{hold}$.

Finally, the drop-out current (see above and Fig. 4) may be included as another data point for the backward pass. The drop-out position corresponds with $I_{drop}$. That is, $I_{B-null}(X_{drop}.) = I_{drop}$.
referred to as the "pull-out current," as depicted in Fig. 6. The value of the $I_{B-null}(X_{closed})$ may be estimated via a linear interpolation based on the calculated $I_{B-null}$ values closest to $X_{closed}$, (typically, X=0). For example, the estimated value of the pull out current may be included as another data point. It may be estimated as follows:

$$I_{B-null}(X_{closed}) = I_{B-null}[6] + \frac{(I_{B-null}[7] - I_{B-null}[6]) * (X_{closed} - X_{null}[6])}{(X_{null}[7] - X_{null}[6])}$$

For each of the forward pass and the backward pass, a best fit curve may be determined. These best fit curves may be calculated from a single trial or from multiple trials. As an example, the polynomials representing best fit curves for $I_{F-null}(X)$ and $I_{B-null}(X)$ are written above the graph of Fig. 8. The coefficients from each of these best fit curves may be stored in the EEPROM as representative of $I_{F-null}(X)$ and/or $I_{B-null}(X)$ in the calibration data. Alternatively, a table of values generated from these best fit curves may be stored in the EEPROM as representative of $I_{F-null}(X)$ and/or $I_{B-null}(X)$ in the calibration data.

*di/dF CALIBRATION*

Due to, *inter alia,* friction between the physical elements of a solenoid actuator, the di/dF at a particular position may be different depending on the direction in which the armature is moving. The armature may be considered to be moving forward when the valve is opening (i.e., the armature is moving against the force of the spring). The armature may be considered to be moving backward when the valve is closing (i.e., the armature is moving with the force of the spring). That is, in this calibration embodiment, di/dF(X) may have a forward value and a backward value for each value of X. di/dF$_F$(X) may be understood to represent the forward di/dF value as a function of armature position. di/dF$_B$(X) may be understood to represent the backward di/dF value as a function of armature position. (It should be noted that di/dF$_F$(X) and di/dF$_B$(X) are estimations, but are sufficient for the purposes of embodiments disclosed herein. In addition to being functions of X, di/dF$_F$ and di/dF$_B$ are also functions of the temperature of the valve, the current flowing through the valve, friction, and the velocity of the armature.)

Fig. 6 also depicts calibration measurements taken with respect to a particular solenoid pinch valve that may be used to determine di/dF$_F$(X) and di/dF$_B$(X), as well as other calibration values. The data in Fig. 6 includes data from a single trial, which includes a "forward pass" and a "backward pass."

Measured values for data points 1-10 may be determined in the same manner as described above with respect to calibrating null current. Moreover, the functions of di/dF$_B$(X) and di/dF$_F$(X) may be derived from the same measured values as $I_{F-null}(X)$ and $I_{B-null}(X)$ may be derived from. Thus, both di/dF calibration values and null current calibration values may be derived from the same set of calibration measurements for a given solenoid valve.

For each data point, #, a di/dF value may be calculated. This may be denoted as di/dF [#]. Further, for each data point #, $X_{null}[#]$ is calculated. Because di/dF [#] may be derived from the same data points as described above with respect to the calculation of $I_{null}[#]$, the calculated values $X_{null}[#]$ for $I_{null}[#]$ may be understood to correspond with di/dF [#]. Thus, for each data point #, di/dF[#] is calculated to correspond with the null position. Further, this di/dF[#] may be understood to be either di/dF$_F$ ($X_{null}[#]$) or di/dF$_B$($X_{null}[#]$), depending on whether the data point is from a "forward pass" or "backward pass," respectively. For example, where # is a "forward pass" data point, di/dF [#] = di/dF$_F$($X_{null}[#]$). Similarly, where # is a "backward pass" data point, di/dF [#] = di/dF$_B$ ($X_{null}[#]$). That is, both di/dF$_F$ and di/dF$_B$, may be considered to be functions of X, where X=$X_{null}$.

For each data point, a value of di/dF [#] may be determined by following formula:

$$di/dF[\#]= -\frac{(I[\#z] - I[\#y])}{(F[\#z] - F[\#y])}$$

For example, for data point 1, di/dF [1] may be calculated as follows:

$$di/dF[1]= -\frac{(I[1z] - I[1y])}{(F[1z] - F[1y])}$$

And because di/dF [1] was calculated from a "forward pass" data point, it may be understood to be $di/dF_F$.

Similarly, for data point 6, di/dF [6] may be calculated as follows:

$$di/dF[6]= -\frac{(I[6z] - I[6y])}{(F[6z] - F[6y])}$$

And because di/dF [6] was calculated from a "backward pass" data point, it may be understood to be $di/dF_B$.

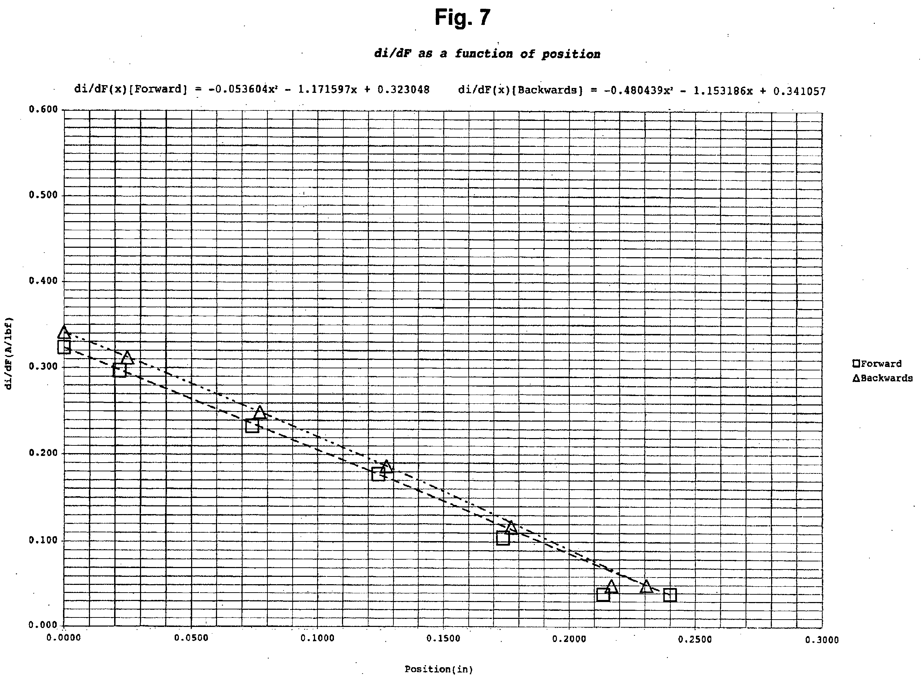

As depicted in Fig. 7, once di/dF is calculated for each data point, those values-for the forward pass and the backward pass-may be graphed. A best fit curve may be determined with respect to di/dF for each of the forward pass and the backward pass.

Due to mechanical limitations of this calibration technique, current and force calibration measurements cannot be taken when the solenoid pinch valve is fully open or fully closed. Because current and force calibration measurements cannot be taken in the fully open and fully closed positions, di/dF at these positions cannot be calculated from such data. Moreover, a best fit curve (and its polynomial representation) might not be accurate at points outside of the range of data used for the best fit curve calculation. Because of this, estimating di/dF at fully open or fully closed positions via a best fit curve may yield inaccurate results. Thus, it may be advantageous to estimate di/dF at open or closed positions-$di/dF_F(X_{hold})$, $di/dF_B(X_{drop})$, $di/dF_F(X_{closed})$, and $di/dF_B(X_{closed})$, respectively-via geometrical interpolation based on the data points closest to $X_{hold}$, $X_{drop}$ and $X_{closed}$, or simply by taking di/dF values of positions closest to the end points.

$di/dF_F(X_{closed})$ may be estimated from data points 1 and 2 based on the following equation.

$$di/dF_F(X_{closed})[1,2]= di/dF[1] + \frac{(X_{closed} - X_{null}[1]) * (di/dF_F[1] - di/dF_F[2])}{(X_{null}[1] - X_{null}[2])}$$

To increase accuracy, additional $di/dF_F(X_{closed})$ data points may be calculated based on measurements from additional trials in a similar manner. Because $di/dF_F(X_{closed})$ is calculated from forward pass measurements, and because it is associated with the initial opening of solenoid pinch valve, the estimated $di/dF_F(X_{closed})$ value may be used to determine the best fit curve for $di/dF_F$.

$di/dF_B(X_{closed})$ may be estimated from data points 6 and 7 based on the following equation:

$$di/dF_B(X_{closed})[6,7]= di/dF[6] + \frac{(X_{closed} - X_{null}[6]) * (di/dF_B[6] - di/dF_B[7])}{(X_{null}[6] - X_{null}[7])}$$

To increase accuracy, additional $di/dF_B(X_{closed})$ data points may be calculated based on measurements from additional trials in a similar manner. Because $di/dF_B(X_{closed})$ is calculated from backward pass measurements, and because it is associated with the closing of solenoid pinch valve, the estimated $di/dF_B(X_{closed})$ value may be used to determine the best fit curve for $di/dF_B$.

$di/dF_F(X_{hold})$ may be estimated by simply adopting the $di/dF_F$ value closest to $X_{hold}$. That is, with reference to Fig. 6, $di/dF_F(X_{hold}) \approx di/dF_F(X_{null}[5])$. This estimate method is used rather than interpolation because the feedback calculation risk of inadequate current compensation is much greater when di/dF is underestimated, as opposed to when di/dF is overestimated. To increase accuracy, additional $di/dF_F(X_{hold})$ data points may also be estimated based on measurements from additional trials in a similar manner. Because $di/dF_F(X_{hold})$ is calculated from forward pass measurements, and because it is associated with the opening of solenoid pinch valve, the estimated $di/dF_F(X_{hold})$ value may be used to determine the best fit curve for $di/dF_F$. This best fit curve for $di/dF_F(X)$ and its representative polynomial are shown in Fig. 7.

$di/dF_B(X_{drop})$ may be estimated by simply adopting the $di/dF_B$ value closest to $X_{drop}$. That is, with reference to Fig. 6, $di/dF_B(X_{drop}) \approx di/dF_B(X[10])$. This estimate method is used rather than interpolation because the risk of inadequate current compensation is much greater when di/dF is underestimated, as opposed to when di/dF is overestimated. To increase accuracy, additional $di/dF_B(X_{drop})$ data points may also be estimated based on measurements from additional trials in a similar manner. Because $di/dF_B(X_{drop})$ is calculated from backward pass measurements, and because it is associated with the initial closing of solenoid pinch valve, the estimated $di/dF_B(X_{drop})$ value may be used to determine the best fit curve for $di/dF_B$. This best fit curve for $di/dF_B(X)$ and its representative polynomial are shown in Fig. 7.

The coefficients from each of these best fit curves may be stored in the EEPROM as representative of $di/dFp(X)$ and/or $di/dF_B(X)$ in the calibration data. Alternatively, a table of values generated from these best fit curves may be stored in the EEPROM as representative of $di/dF_F(X)$ and/or $di/dF_B(X)$ in the calibration data. In another embodiment, a table of values or best curve coefficients could be stored for $dF/di_F(X)$ and/or $dF/di_B(X)$, which could be calculated from $di/dF_F(X)$ and/or $di/dF_B(X)$, respectively.

## $F_{TRIM}$ CALIBRATION

Described below are methods to measure Work performed during the closing of a solenoid valve. However, aside from the compression of tubing, the actual work performed by the armature actuation is not directly relevant to tube detection. For example, work performed by the armature due to friction within the solenoid valve or the valve's spring-though perhaps virtually negligible-is not indicative of any work done to compress tubing. This non-tube compressing work can effectively be excised from the Work calculation through the use of an $F_{TRIM}$ value. $F_{TRIM}$ may be expressed in units of force (e.g., work divided by distance). For the reasons discussed above, in preferred embodiments, a different $F_{TRIM}$ calibration value may be associated with each individual valve. (In alternative embodiments, an $F_{TRIM}$ value may be associated with a class of valves.)

$F_{TRIM}$ may be calculated as follows: First, the amount of Work associated with closing a tubeless valve may be calculated via the methods described below. Then, the total amount of Work may be divided by the valve's stroke length to yield $F_{TRIM}$. In some embodiments, the amount of Work associated with closing a tubeless valve may be determined multiple times and then averaged before being divided by the stroke length. In a preferred embodiment, the $F_{TRIM}$ value is then stored in an EEPROM or other memory device associated with the particular valve.

## SYSTEMS AND METHODS OF TUBE DETECTION

In order to detect the presence of a tube, systems and methods consistent with the disclosed embodiments may sum up work, W, as the solenoid pinch valve closes. Because, in at least one embodiment, gravity has a negligible effect on the armature, the work completed, W, may be assumed to represent the work done upon the tube by the armature. Velocity and gravity assumptions are discussed below.

In preferred embodiments, the tube detection method is effectuated while the valve is closing, and thus the backward pass data is used. Further, in some embodiments, this tube detection method may be used each time the valve closes.

In an embodiment pertaining to a solenoid pinch valve that is also an intelligent fluid control component, the best fit curve polynomial coefficients for one or more of $di/dF_B(X)$, $di/dF_F(X)$, $I_{B-null}(X)$, and $I_{F-null}(X)$ are stored in the EEPROM coupled to the solenoid pinch valve. This data could also be stored in the EEPROM as tables of values. However, storing the data as coefficients may be a more efficient use of EEPROM memory. Alternatively, some or all of this data could be stored elsewhere, such as in the controller software or in other memory.

As an illustration, when provided with data representative of $di/dF_B(X)$-for example in the form of polynomial coefficients-the controller may calculate corresponding values for $dF/di_B(X)$ and store those values in a table in controller software. Such a table may provide values for $dF/di_B$ indexed by armature position, X.

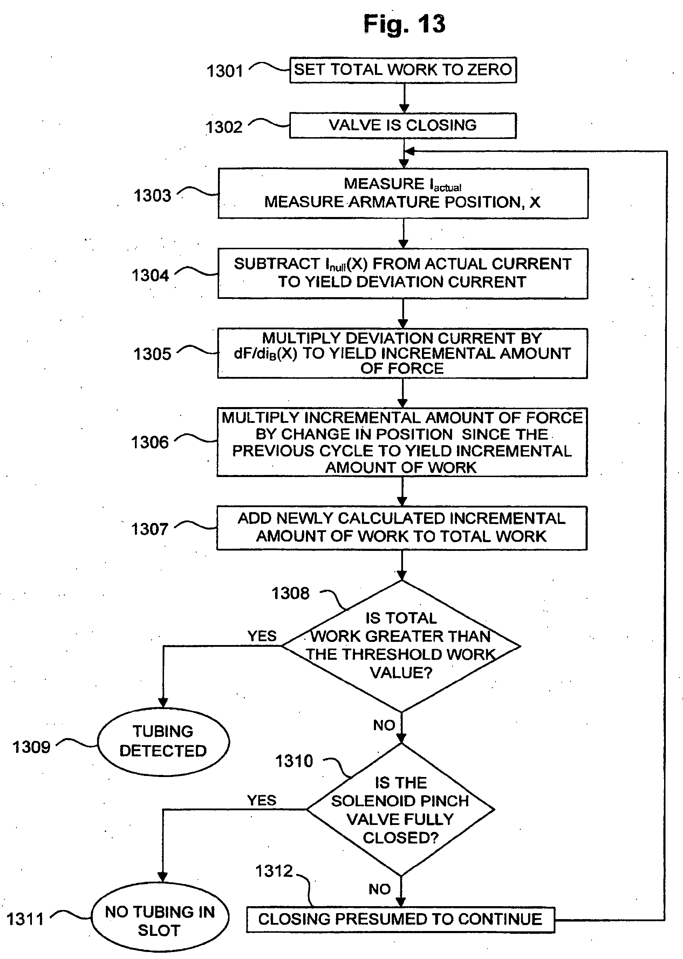

A controller may control a fluid control system by continuously cycling through a higher level controller software function. Some embodiments of this method are described below and a preferred embodiment is depicted as a flowchart in Fig. 13. First, as shown in steps 1301 and 1302, the value for total work is set to zero before the measurement cycle begins and the valve is closing. For each cycle of the controller software, the controller may determine electrical and other characteristics pertaining to each connected fluid control component. For example, in each cycle, the controller may determine the actual current flowing through a solenoid pinch valve-$I_{actual}$-and the position of the armature-X, as in step 1303. This data may be used by the tube detection method. And in one alternative embodiment, the tube detection method may not begin summing amount of work completed-W-until the armature has reached a steady velocity (as determined by, for example, comparing successive position measurements).

Because the effect of gravity is assumed to be negligible (or is otherwise compensated for), it may be assumed that any deviation ($\Delta I$) of the measured current from the null current is the result of force being exerted on the armature by a tube (or of noise in the system). Therefore the deviation current, $\Delta I$ may be determined as follows, as in step 1304.

$$\Delta I = (I_{actual} - I_{B\text{-}null}(X))$$

Thus, as in step 1305, multiplying this deviation in current by the appropriate dF/di value should yield the incremental amount of force being exerted-ΔF:

$$\Delta F = \Delta I * dF/di_B(X)$$

In another embodiment, the incremental amount of force being exerted may be modified by using $F_{TRIM}$ as follows:

$$\Delta F = \Delta I * dF/di_B(X) + F_{TRIM}$$

Adding $F_{TRIM}$ to the incremental amount of force, effectively excises work done by valve in closing that is not associated with the compression of tubing (e.g., work associated with friction within the solenoid and gravity). That is, where no tubing is present, W, when calculated using $F_{TRIM}$ should reflect an approximate value of 0.. The $F_{TRIM}$ technique is not shown in Fig. 13.

As such, this method (either with or without using the $F_{TRIM}$ value) can determine the incremental amount of work exerted upon an inserted tube (if any) by multiplying the incremental amount of force-ΔF-by the incremental change in position-ΔX-using the following formula, as in step 1306.

$$\Delta W = \Delta F * \Delta X$$

ΔX, the incremental change in position, may be determined by subtracting the current armature position from the armature position in the previous cycle.

Because it is assumed that gravity is already accounted for in the calibration (or is negligible) and that no other forces act upon the armature, W represents the amount of work done by the armature to compress the tube (if any). In one embodiment, the amount of work may be iteratively summed up as each new ΔW is determined, as in step 1307. Then, W may be compared to a threshold value to determine whether or not the armature has compressed a tube in each software cycle. If W is greater than (or equal to) the threshold value, the method will detect a tube, as in step 1309 and the process is completed. If W does not exceed (or meet) the threshold value, a determination is made as to whether the solenoid valve is fully closed, as in step 1310. If it has closed, the determination that no tubing is present in the tube slot is made and the process is completed, as in step 1311. If the solenoid valve has not fully closed, the closing continues, as in step 1312, and the detection cycle repeats, as in steps 1303-1308.

However, where W is less than (or equal to) the threshold value-and the solenoid pinch valve has fully closed, the system may determine that the solenoid pinch valve does not have a tube in place, as in step 1311. In response, the system may call particular software functions that alert the user or otherwise address the lack of tubing, perhaps including system shut down, or electronic notification of other systems.

In another embodiment (not shown in Fig. 13), W may be compared to a threshold value only after the solenoid pinch valve has fully closed.

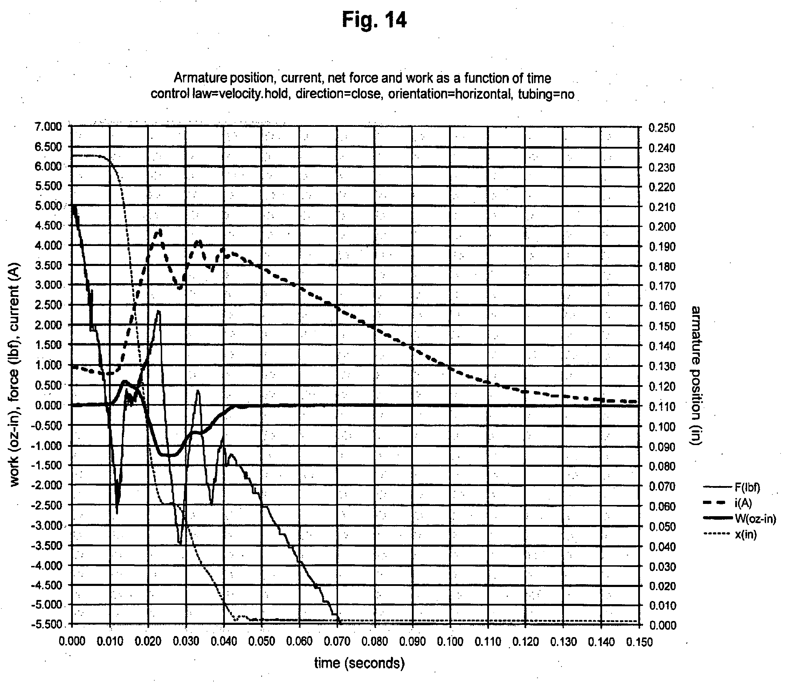

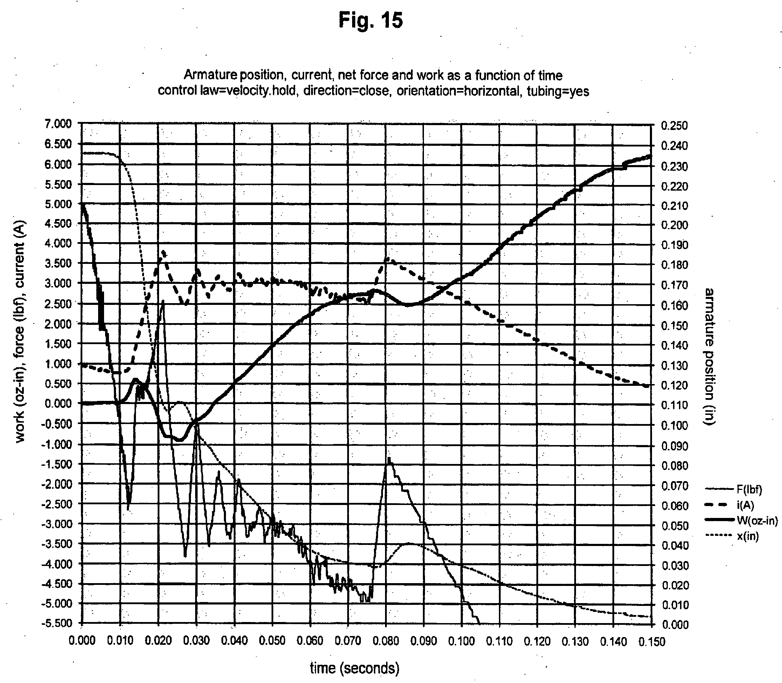

By way of demonstration, Fig. 14 illustrates work involved in the closing of a solenoid pinch valve without tubing. As can be seen from Fig. 14, from the initiation of the closing routine to the end, the net work completed is 0 oz-in. (0 kgf*m) By contrast, Fig. 15 illustrates work involved in the closing of a solenoid pinch valve with tubing. As can be seen from Fig. 15, from the initiation of the closing routine to the end, the net work completed is 6.25 oz-in (0.0045 kgf*m).

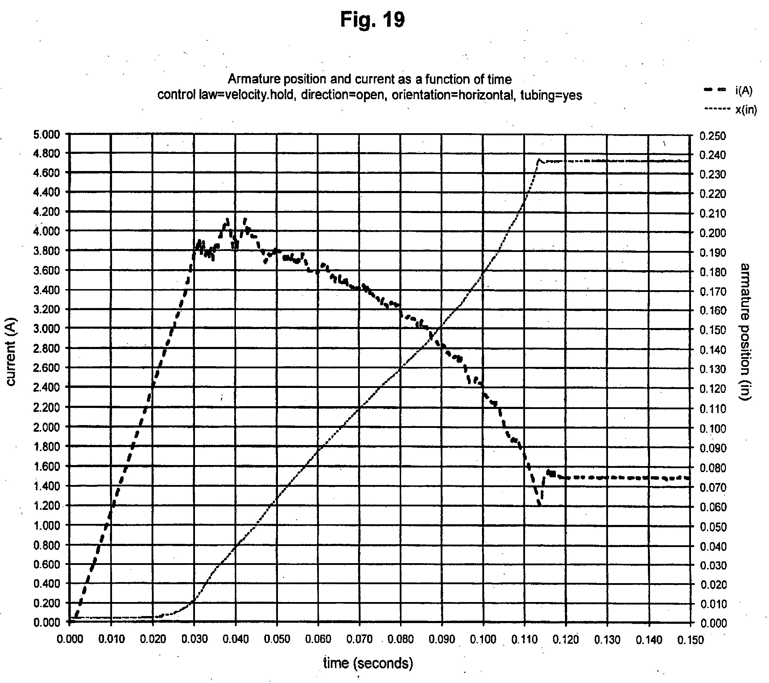

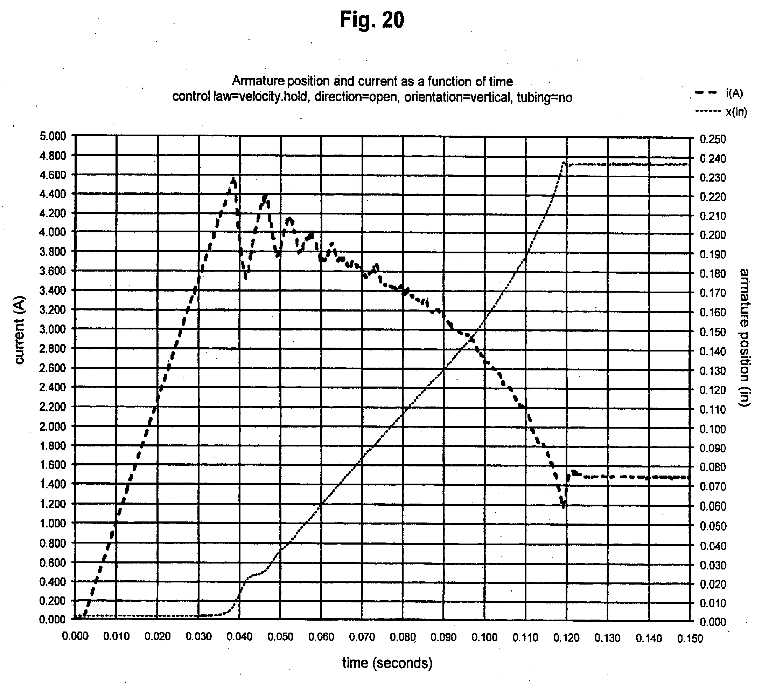

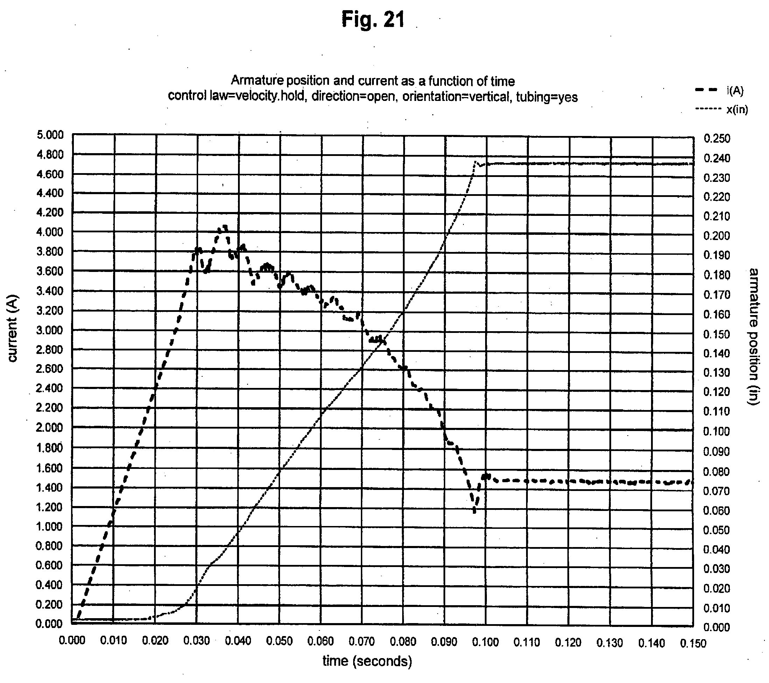

The data depicted in Figs. 14- 21 was captured using an FCP-A1 controller from an Acro 2400 solenoid pinch valve. Where the figures reflect tubing inserted into the valve, the tubing was 0.390" (9.9 mm) O.D. silicone tubing.

These figures also demonstrate the effectiveness of tube detection method embodiments wherein work is not measured until the armature has a steady velocity. For example, Fig. 14 illustrates work involved in the closing of a solenoid pinch valve without tubing. As can be seen from Fig. 14, the armature reaches a steady velocity by approximately 0.015 seconds, wherein completed work is approximately 0.5 oz.-in. (0.00036 kgf*m) From that point, the work completed as the solenoid pinch valve closes is approximately -0.5 oz.-in. (-0.00036 kgf*m) (0 oz.-in. - 0.5 oz-in = -0.5 oz.-in. (0 kgf*m - 0.00036 kgf*m = -0.00036 kgf*m)). By contrast, Fig. 15 illustrates work involved in the closing of a solenoid pinch valve with tubing. As can be seen from Fig. 15, the armature reaches a steady velocity by approximately 0.015 seconds, wherein completed work is approximately 0.5 oz.-in (0.00036 kgf*m), From that point, the work completed as the solenoid pinch valve closes is approximately 5.75 oz.-in. (0.00414 kgf*m) (6.25 oz.-in. - 0.5 oz.-in. = 5.75 oz.-in. (0.0045 kgf*m - 0.00036 kgf*m = 0.00414 kgf*m)).

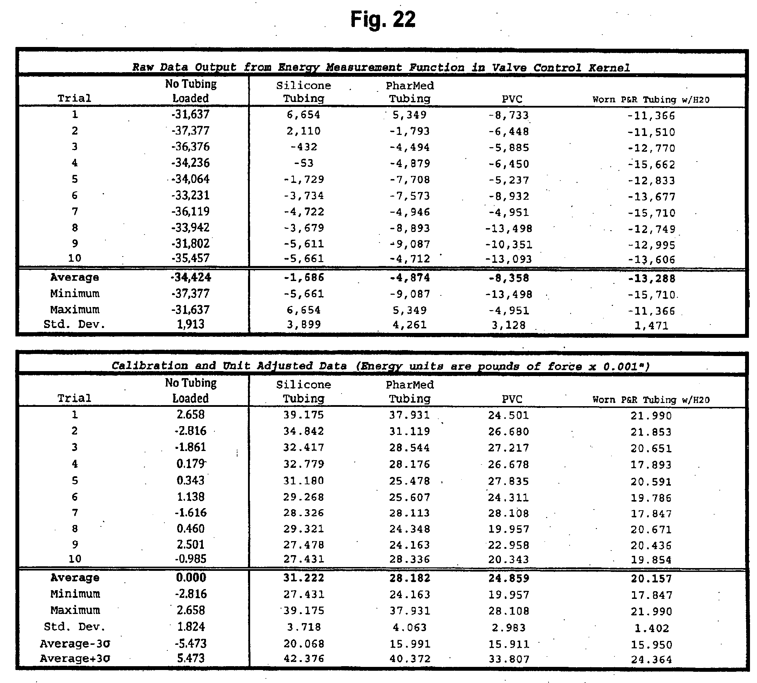

Figure 22 illustrates the different amounts of work required to compress various tubing samples by a particular solenoid pinch valve (Acro Model No. 2300-H-AB-V105). (The solenoid pinch valve used to determine the sample values in Fig. 22 is of a different model than the solenoid pinch valve used in all other Figures and examples. The valve used to determine the sample values in Fig. 22 is a 2300 valve with a stroke length of approximately 0.150 inches (3.81 mm). Nonetheless, Fig. 22 illustrates the effectiveness of the tube detection technique regardless of the type of tubing inserted into the valve.) The upper table expresses work required as raw data output from controller software. The lower table expresses work required in energy units of pounds of force * 0.001 inches (0.0000115 kgf*m). In this lower table, the work required to compress each tubing sample, respectively, is offset by the amount of work calculated when closing a tube-less valve. The magnitude of threshold value should be significantly less than the magnitude of work required to compress the softest tubing. Further, the magnitude of threshold value should be significantly greater than the magnitude of work required to close a tube-less valve. As such, the tube detection method should be fairly robust, having large tolerances built into the detection algorithm. For example, with respect to the solenoid pinch valve depicted in Figure 22, the threshold work value may be selected by taking the midpoint of two values: (a) the average + $3\sigma$ of the work required to close the valve with no tube inserted and (b) the average - $3\sigma$ of the work required to close the valve with the weakest, most malleable tube inserted into the valve. In other embodiments, additional measurements could be taken for various tubes in various positions with respect to gravity, such where a solenoid pinch valve is oriented vertically. A more robust tube detection method may result from determining a work threshold value from a larger sample size, with respect to types of tubes, wear of tubes, age of tubes, contents of tubes, and positions of the solenoid pinch valve.

In other embodiments, the threshold value used in the above described calculation can be more narrowly tailored for a particular type of tubing that is expected to be used with the solenoid valve. This type of threshold value may be associated with a particular solenoid valve type-tubing type combination.

## ADDITIONAL DISCUSSION OF, USES OF, OR IMPROVEMENTS TO TUBE DETECTION METHODS AND SYSTEMS

### DETERMINING PRESSURE

Once a valve is closed, the W value may be understood to represent the work required to compress a tube. As such, the W value may serve as an analog estimate of the pressure of fluid inside a tube. Similarly, W may be used to classify a tube by its hardness, which may, in turn, permit a controller to automatically identify the particular type of tubing present within a solenoid pinch valve. Such a classification may be achieved by providing multiple threshold comparison values for different tubing types and/or fluid pressures.

### THE GRAVITY ASSUMPTION

Typically, when a solenoid pinch valve is calibrated, it may be positioned horizontally with respect to the ground. That is, when a solenoid valve is oriented parallel to the ground, gravity does not act on the armature to either close it or open it. (Ground typically references the earth, but, for example, in the case of a space vehicle "ground" may refer to virtually any gravity source or sources.) Rather, aside from creating some friction between the armature and the rest of the valve, gravity does not measurably affect the valve's operation. As such, when a solenoid pinch valve is oriented parallel to the ground during its operation, it may be assumed that gravity does not substantively affect the work done by the valve.

However, when a solenoid pinch valve is oriented perpendicularly to the ground (or at some non-zero angle with respect to the ground), the mass of the armature may affect the valve's operation. For example, if a solenoid pinch valve is oriented so the armature approaches the ground as it closes, the force of gravity acts to close the pinch valve. Similarly, if a solenoid pinch valve is oriented so the armature approaches the ground as it opens, the force of gravity acts to open the pinch valve.

A non-horizontal orientation of a solenoid pinch valve is unlikely to hinder the ordinary operation of a pinch valve, such as opening and closing. However, solenoid valve operations that rely upon on sensitive calibration measurements (and calculations), such as electronic tube detection and electromechanical damping, may potentially be hindered by such a valve orientation with respect to ground. That is, a valve is typically calibrated in a horizontal orientation, and the calibration is predicated on the assumption that gravity will act upon the armature in the same manner.

However, in one embodiment, the effect of gravity on the work calculation may simply be ignored. In such an embodiment, the threshold work value calculation should (but need not necessarily) factor in various orientations. For example, Fig. 14 illustrates work involved in the closing of a horizontally-oriented solenoid pinch valve without tubing. As can be seen from Fig. 14, from the initiation of the closing routine to the end, the net work completed is 0 oz-in. (0 kgf*m) By contrast, Fig. 15 illustrates work involved in the closing of a horizontally-oriented solenoid pinch valve with tubing. As can be seen from Fig. 15, from the initiation of the closing routine to the end, the net work completed is 6.25 oz-in. (0.0045 kgf*m)

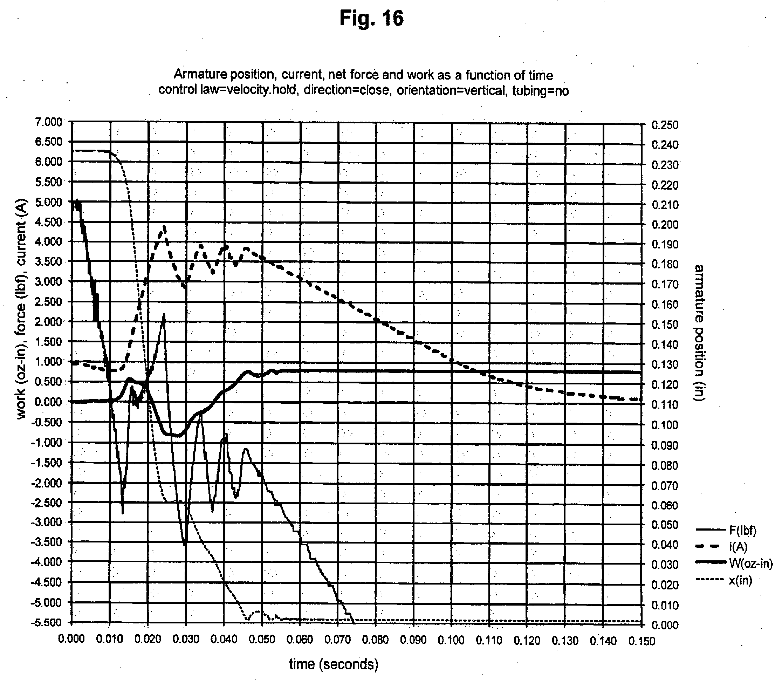

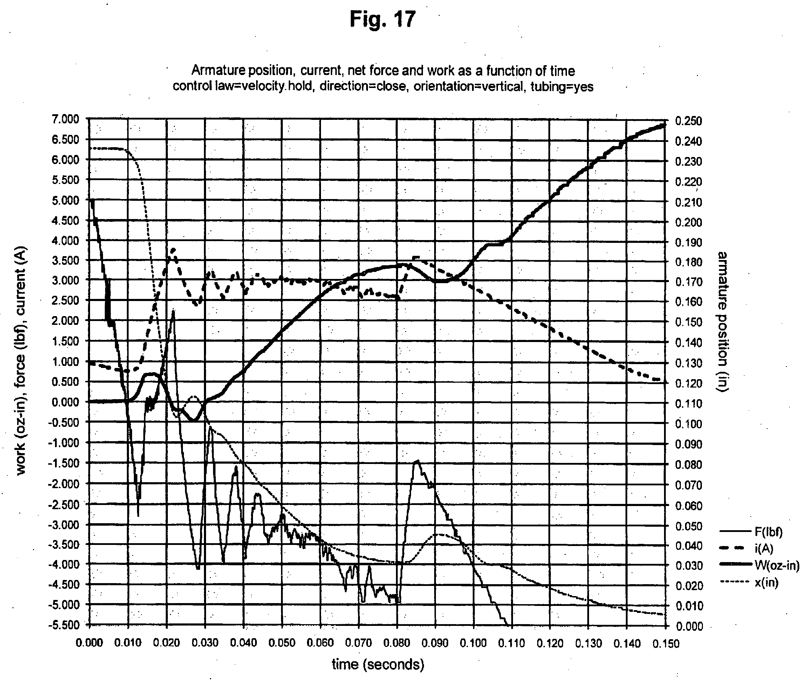

In obtaining the data shown in Figs. 16 and 17, the solenoid pinch valve was oriented vertically such that gravity works against closing the valve. And with respect to vertically oriented valves, Fig. 16 illustrates work involved in the closing of a solenoid pinch valve without tubing. As can be seen from Fig. 16, from the initiation of the closing routine to the end, the net work completed is 0.75 oz-in. (0.0086 kgf*m) (The approximate 0.75 oz-in (0.0086 kgf*m) difference in net work between Fig. 16's net work and that of Fig. 14 is largely due to gravity.) By contrast, Fig. 17 illustrates work involved in the closing of a vertically oriented solenoid pinch valve with tubing. As can be seen from Fig. 17, from the initiation of the closing routine to the end, the net work completed is 6.90 oz-in. (0.7950 kgf*m) (The approximate 0.65 oz-in. (0.00749 kgf*m) difference in net work between Fig. 17's net work and that of Fig. 15 is largely due to gravity.)

Thus, for this particular valve and tubing, the range of potential work threshold values for which tubing would be accurately detected is the range between 0.75 oz-in (0.0086 kgf*m) (no tubing in a vertical valve) to 6.25 oz-in (0.7950 kgf*m) (tubing in a horizontal valve). Selection of a threshold work value in the center of this range should permit a robust tube detection without regard to gravity (and virtually without regard to the type of tubing).

In another embodiment, the operational position of a valve can be compensated for via the initial calibration. That is, if the solenoid pinch valve is oriented (with respect to the ground) during the calibration process in the same manner as it is oriented during use, the effect of gravity on the mass of the armature will be "included" in the calibration values and calculations. In other words, the effect of gravity on the solenoid pinch valve will be taken into account by the calibration. Using an $F_{TRIM}$ value in the calculation of $\Delta F$ (and, therefore, W) may be used to effectuate this embodiment.

In yet another embodiment, one or more accelerometers, orientation sensors, or other MEMs device coupled to the solenoid pinch valve can be used to determine the orientation of the valve. When connected to the controller, such an orientation sensor (or sensors) can be used to notify a fluid control system of a non-horizontal orientation of a fluid control component, as well as information relating to its orientation. The controller may use data from the orientation sensor to, for example, select calibration values (e.g., if the EEPROM contains multiple sets of calibration values taken at various valve orientations), recalculate calibration values based upon the force of gravity (or other forces affecting the orientation sensor and valve), modify the calculation of W to accommodate gravity, or otherwise adjust the controller software. For example, in one embodiment, several $F_{TRIM}$ calibration values corresponding to several orientations with respect to the ground, respectively, may be stored as calibration data. And the orientation of a valve with respect to gravity can be accurately determined by means of a two-axis accelerometer as would be understood by a person of skill in the art. In other embodiments, the orientation of the valve with respect to gravity can be accurately determined by a single axis accelerometer, provided that the accelerometer's axis is aligned with the axis of the armature. In this embodiment, the $F_{TRIM}$ value associated with the orientation of the valve during its actual use will be the value utilized in the calculation of $\Delta F$, and, in turn, W.

In yet another embodiment, the effect of gravity can be mathematically excised from the calculation of W. When a pinch valve closes on tubing, the tubing acts like a spring. That is, the force that the tubing exerts on a valve's armature increases, at least theoretically, in a linear manner as the tube is compressed. The force of gravity, however, may be assumed to act upon the armature in a constant manner. Thus, by measuring the force acting upon the armature once the armature reaches a constant velocity, and prior to any possible interaction with a tube, the force on the armature due to gravity-$F_g$-may be determined. As such, during each cycle of the tube detection method (wherein $\Delta W$ is calculated), work due to gravity may be excluded in accordance with the following formula:

$$\Delta W = (\Delta F + F_g) * \Delta X$$

Then, as work is summed up, the effect of gravity will be mathematically removed.

*THE VELOCITY ASSUMPTION*

In one embodiment, velocity may be "assumed" to be constant despite a changing velocity. That is, the work associated with accelerating an armature-especially an armature with a small mass-is significantly less than the work associated with compressing tubing with the armature. As such, any work done to accelerate the armature may be considered negligible when compared to the work done to compress the tubing.

Moreover, the net change in velocity of the armature over the entire closing process (i.e., comparing the velocity when closing begins to when closing ends) is zero. That is, the threshold work value should have sufficient tolerance such that any work done by the acceleration (including deceleration) of the armature will not affect the ultimate determination as to whether or not a tube is detected. In other words, the velocity assumption can be ignored altogether in some embodiments. Indeed, the measurement of W may begin as soon as the valve begins to close.

In another embodiment, velocity may be assumed to be constant because the summing of work begins only after the armature has accelerated to its target velocity, and because the solenoid valve may be operated with an electromechanical damping algorithm in order to maintain the target velocity. U.S. Patent Application Publication Nos. 2010/020074 A1 and 2010/0188667 A1 describe a method to substantially meet and maintain a target armature velocity via electromechanical damping by using position measurements and a fixed gain control based on $I_{null}$ (therein referred to as $I_{non-acceleration}$). Further, as disclosed below, the electromechanical damping method described in U.S. Patent Application Publication Nos. 2010/020074 A1 and 2010/0188667 A1 may be enhanced by utilizing di/dF data in a revised algorithm:

### IMPROVED <u>ELECTROMECHANICAL</u> DAMPING

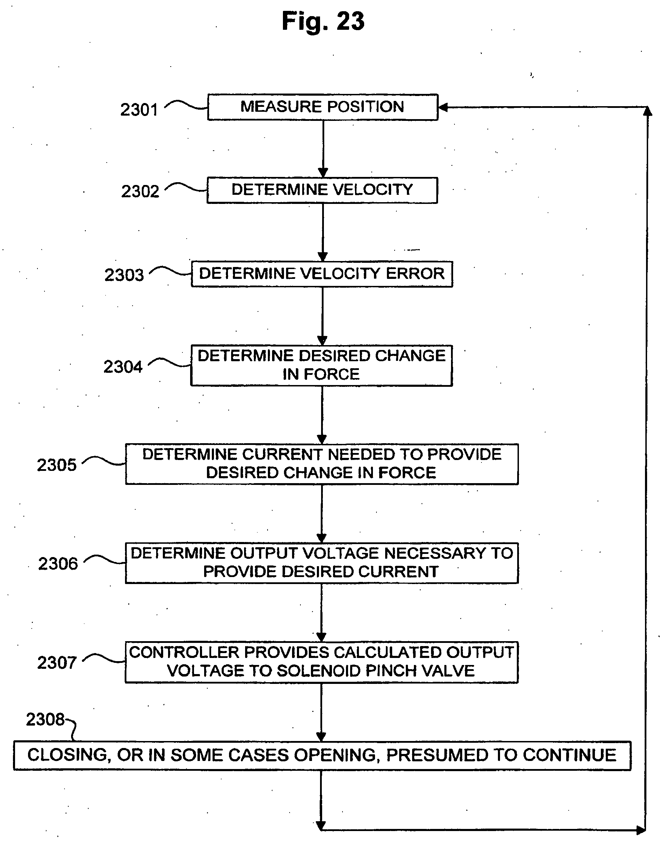

As described in U.S. Patent Application Publication Nos. 2010/020074 A1 and 2010/0188667 A1, electromechanical damping may be effectuated by using a single gain value and a table of $I_{null}$ (therein referred to as $I_{non-acceleration}$) values to produce an adjustment current. This method may be improved by utilizing a variable gain, because, electrically, a solenoid pinch valve is a non-linear system. Some embodiments of this method are described below and depicted in the flow chart of Fig. 23: These di/dF damping embodiments may be used to control the velocity of the solenoid armature. For example, as described below, a constant velocity may be achieved and maintained using such a technique.

First, the algorithm may measure armature position, as in step 2301, and determine actual velocity, $Vel_{act}$, as in step 2302. $Vel_{act}$ may be determined by subtracting the current armature position from its position in the previous cycle (e.g., 40 microseconds prior). In some embodiments, this change in position over a single cycle, may serve as $Vel_{act}$ (also $\Delta X$, as discussed above). In other embodiments, $Vel_{act}$, the change in position may be divided by the cycle time to determine a $Vel_{act}$ value with traditional velocity units. Next, the algorithm may determine the velocity error-$\Delta Vel$, as in step 2303. This is determined by subtracting the actual velocity-$Vel_{act}$-from the desired (set point) velocity-$Vel_{set}$.

$$Vel_{set} - Vel_{act} = \Delta Vel$$

Then, the velocity error is multiplied by the force gain to determine the desired change in force, as in step 2304. This force gain value is determined through modeling or experimentation for each type of valve. For example, a small valve with a stroke of 0.150 inches (3.81 mm) and an armature mass of 100g (0.2205 lb.) might have a force gain of 0.5 lb. * second / inch (0.0089 kg*s/mm). Thus, the desired change in force $\Delta F$, may be determined through the following equation:

$$\Delta Vel * ForceGain = \Delta F$$

Then, as in step 2305, $\Delta F$ may be used to find the desired new current-$I_{setpoint}$-by the following equations:

$$\Delta F * di/dF(X) = \Delta I$$

$$I_{setpoint} = I_{null(X)} + \Delta I$$

If this velocity is being controlled as the pinch valve is closing, then $di/dF_B(X)$ is used in the above equation. However, if this velocity is being controlled as the pinch valve is opening, then $di/dF_F(X)$ is used.

Once the desired $I_{setpoint}$ is determined, another control loop may be used to achieve $I_{setpoint}$ using the voltage output from the controller board into the solenoid pinch valve, as in step 2306. This method uses two modeled or experimentally determined gain coefficients. In one embodiment, the gain coefficients are determined for each model of solenoid valve. That is, individual calibration of gain coefficients for each individual valve unit may not be necessary.

The first gain coefficient is the proportional gain coefficient-KIp. The units for KIp are Volts/Ampere, which may also be expressed as $\Omega$. Typically, KIp is equal to the DC resistance of the solenoid (also in $\Omega$). Otherwise, KIp may be determined empirically.

The second gain coefficient is the differential gain coefficient-$KI_D$. The units for $KI_D$ are Volts*second/Ampere, which may also be expressed as $\Omega$ * second. This gain coefficient is used because the solenoid valve is electrically reactive. $KI_D$ may be determined by testing the valve's step response. $KI_D$ has been appropriately determined when the valve's step response shows critical damping. Use of $KI_D$ in electromechanical damping represents an improvement to previous embodiments because it helps prevent current overshoot. Use of $KI_D$ also reduces current ripple, further reducing noise from stator (solenoid coil) and armature vibration.

In some embodiments, the voltage output of the controller is a PWM (pulse width modulated) signal. Where the voltage output is in this form, this PWM voltage will correspond to the effective output voltage-$V_{out}$-that is calculated herein.

$$[0140] \text{ If } I_{setpoint} = 0 \text{ A, then } V_{out}=0.$$

If $I_{setpoint} \neq 0$ A, then $V_{out}$ is calculated as follows. First, AV is calculated, where $I_{actual}$ is the most recently measured current and $I_{prev}$ is the current measured during the previous cycle.

$$\Delta V = KI_P * (I_{setpoint} - I_{actual}) - KI_D * (I_{actual} - I_{prev})$$

It may be noted that "$(I_{actual}-I_{prev})$" is a representation of di/dt. Then, output voltage may be calculated as follows, where $V_{prev}$ is the voltage output from the previous cycle.

$$V_{out} = V_{prev} + \Delta V$$

It may be noted that above equation may be subject to $0 \leq V_{out} \leq V_{max}$, where $V_{max}$ is the power supply voltage. The controller then outputs $V_{out}$ to the solenoid pinch valve, as in step 2307. Presuming that the valve continues to close (or alternatively, open) as in 2308, the electromechanical damping algorithm may proceed for another cycle. The software function cycles may continue until the solenoid valve is closed, until the solenoid pinch valve has determined that tubing is present (and the armature has at least partially compressed the tubing), or until damping is otherwise no longer desired or required.

The use of the above described electronic damping method in a closing valve is depicted in Figs. 14, 15, 16, and 17. In these embodiments, the method is used to reduce the impact of the armature against its contacting surface by controlling the armature's velocity. It may be observed that this technique may be used during the closing of a solenoid pinch valve regardless of orientation of the valve or the presence of tubing.

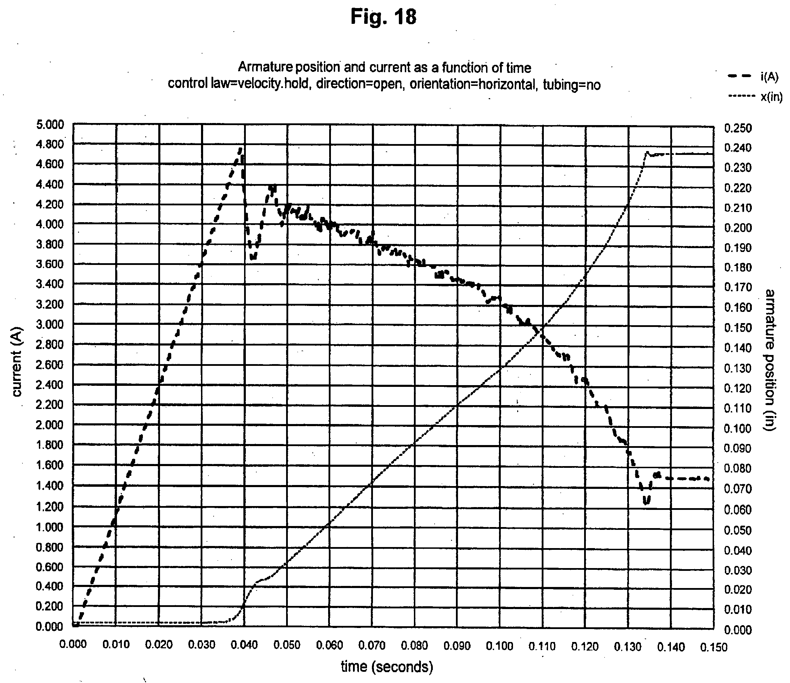

The use of the above described electromechanical damping method in an opening valve is depicted in Figs. 18, 19, 20, and 21. In these embodiments, the method is used to reduce the impact of the armature against the fixed pole of the solenoid pinch valve by controlling the armature's velocity. It may be observed that this technique may be used during the opening of a solenoid pinch valve regardless of orientation of the valve or the presence of tubing.

*RELAX STATE*

As described above, tube detection methods may determine the presence of tubing in the valve before the valve has completely closed. That is, the controller may determine that work, W, is larger than the threshold value before the closing stroke is completed. At this point, because W has met or surpassed the threshold value, it may be understood that the armature is in contact with the tubing. This tubing may be understood to, in part, counteract the force of the spring that drives the valve shut. As such, the tubing will-at least to some degree-slow down the armature, effectuating some physical damping and reducing (or eliminating) the need for further electromechanical damping in that closing stroke.

Thus, in one embodiment, the controller may place the solenoid valve into a "relax state" once W meets or surpasses the threshold value. In the "relax state," the controller may cease (or reduce) electromechanical damping functionality and/or reduce the current that is used to slow the closing of the valve. Without this "relax state" functionality, the tubing may prevent the valve from closing as quickly as desired, or in some extreme instances, may prevent the valve from closing at all. It must be noted, however, that although the tubing may slow the closing of the valve, turning off the current entirely (or reducing it too much) may result in the pinch valve slamming shut.

The effects of the "relax state" software are illustrated in Figs. 14-17. In Figs. 14 and 16, where no tubing is present, "relax state" occurs only when the valve has closed, at approximately 0.042 seconds and 0.047 seconds, respectively. Once "relax state" begins, the current tapers off, and as a result, the force (with respect to the armature-not the force of then tubing) tapers off.

In Figs. 15 and 17, where tubing is inserted into the valve, "relax state" occurs when tubing (and therefore contact with tubing) has been detected, at approximately 0.082 seconds and 0.085 seconds, respectively. Once "relax state" begins the current tapers off, and as a result, the force (with respect to the armature) tapers off.

## *TUBE DISLODGEMENT DETECTION IN A CLOSED SOLENOID PINCH VALVE*

As described above, the presence of tubing may be detected as a solenoid actuator valve closes. However, the above described embodiments may not be easily adapted to detect when tubing within an already closed valve may have been removed since the valve's closing. For example, an equipment malfunction may result in tubing being yanked out of an already closed solenoid valve. The system-having detecting the presence of the tubing as the valve closed-may assume that the tubing remains pinched within the valve and will only detect whether that the tubing became dislodged (e.g., the absence of tubing) upon opening and closing again. In some fluid control applications, the opening and reclosing of the valve might not proceed for seconds, minutes, hours, days, or even longer. As such, the dislodging of pinched tubing from a valve may go unnoticed for a period of time, possibly causing a dangerous condition or otherwise impeding reliable fluid control. Therefore, it is beneficial for a fluid control system to be able to monitor whether tubing may have been dislodged from closed valves.

Generally, tube dislodgement detection in a closed valve may be accomplished by monitoring the movements of the solenoid armature. Such monitoring is preferably accomplished using an optical aperture sensor, but other position sensors (or position sensing methods) may be used. Even after a solenoid valve is sufficiently closed to prevent the flow of fluid through pinched tubing, such a valve may continue to close, the armature further compressing the already compressed tubing (a process sometimes referred to as "settling"). Therefore, any dislodgement detection method should account for the fact that the armature of the solenoid may continue to move after it is closed, despite that tubing may remain properly positioned in the tube slot.

In preferred embodiments, this detection method utilizes indications of the velocity of the armature to determine the amount of armature movement. In preferred embodiments, $Vel_{act}$, as discussed above, is used. To effectuate this tube detection method, the controller may make continuous $Vel_{act}$ measurements over a period of time, for example, 1 millisecond. Thus, for example, if a controller completes a cycle every 40 microseconds, it may take 25 $Vel_{act}$ measurements in 1 millisecond. The controller may take the absolute value (or magnitude) of each $Vel_{act}$, and average them together. The use of a magnitude measurement is preferred because values of $Vel_{act}$ may be positive or negative, and averaging such values together without using their magnitudes would allow positive and negative values to cancel each other out.

In preferred embodiments, the controller continuously calculates a running average of these magnitude $Vel_{act}$ values. That is, for each time period (e.g., 1 millisecond) the running average would be equal to the sum of the magnitudes of the Velar values determined during that time, divided by the number of $Vel_{act}$ magnitude data points (e.g., 25 where the cycle is 40 microseconds). When the next $Vel_{act}$ is determined (e.g., 40 milliseconds later) the running average is updated. This process may run continuously as long as the solenoid valve remains closed. In preferred embodiments, the controller may use a software-based "delay filter" based on a FIFO (first in first out) system to continually produce running average without having to actually recalculate the running average every cycle, thereby saving controller processing power. Utilizing many measurements of $Vel_{act}$ reduces the likelihood that isolated incidents of system noise would falsely indicate a likelihood of tubing dislodgement. In other embodiments, the system may calculate the root mean squared of a set of the $Vel_{act}$ measurements instead of the running average of the magnitudes. In yet other embodiments, a running average is not calculated; rather, a running sum of $Vel_{act}$ magnitude values over the time period may be used.

The running average (or the like) of the $Vel_{act}$ values may be compared to a Relax Maximum Magnitude Threshold value (herein "RMMT"). In one embodiment, RMMT may be expressed in units of distance per second. Because it is a measure of magnitude, it's value may be assumed to be positive.

When the running average is greater than (or perhaps equal to) the RMMT, the system may determine that there is a likelihood that the tubing has been dislodged, and then may send error messages or sound an alert as desired. These error messages may alert the user of a potential problem, initiate failsafe or error modes, or otherwise facilitate verification of and/or remedy for the potential dislodgement.

When the running average is less than (or perhaps equal to) the RMMT, the slow settling of the armature or noise in the system are likely sources of armature movement and the system may assume that the tubing has not been dislodged. The system may therefore continue to monitor the closed valve for tubing dislodgement.

The RMMT value may be determined experimentally for each model of solenoid actuator valve. This is because the RMMT value depends, in large part, on relative strength of the spring that causes the solenoid valve to close in the absence of current. Such an experimental determination may be made by bumping, moving, pulling out, or jiggling the tubing in a closed valve at various RMMT values as a tubing dislodgement detection method is effectuated. With a proper RMMT value, the system should indicate a likelihood of tube dislodgement when the tube is removed or otherwise yanked out of proper position, but should indicate that the tube remains present when the solenoid valve containing the tube is merely bumped or jostled. As one example, a preferred RMMT for a 2400 series Acro valve (designated for 3/8 inch (9.525 mm) tubing) is 1 inch/second 25.4 mm/s).

Additionally, in preferred embodiments, the detection method does not begin until a period of time passes after the valve has closed. This period of time may be called "blanking time" and serves the prevent extraneous motion of the armature resulting from the closing of a solenoid valve from erroneously indicating that the tubing has been removed. In a preferred embodiment, the blanking time may be between 50 milliseconds and 100 milliseconds.

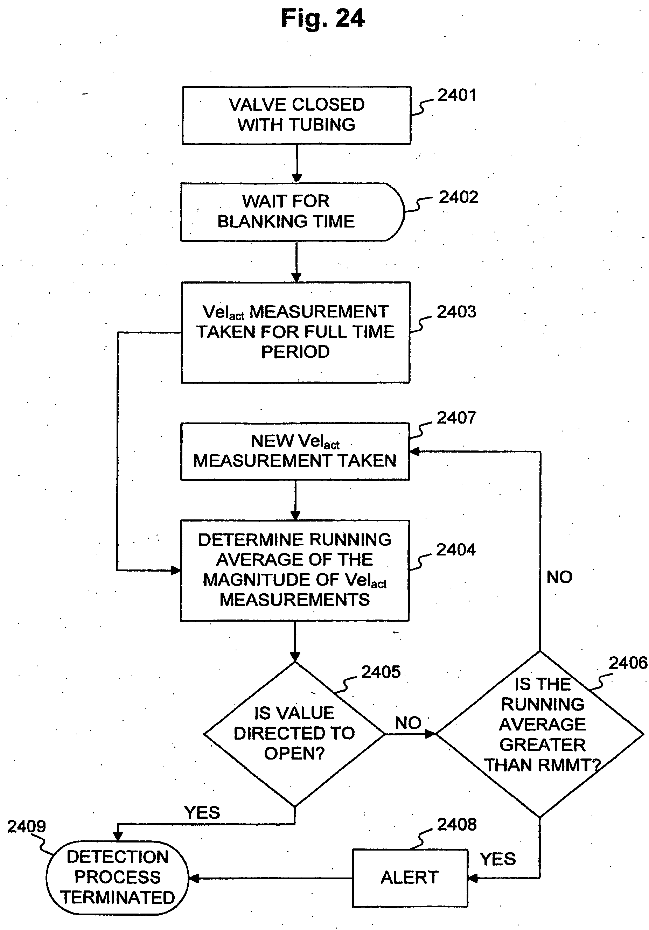

As illustrated in Figure 24, the following steps may be taken to effectuate tube detection in a closed solenoid pinch valve. First, as shown in step 2401, the valve is closed, pinching tubing such that fluid flow is stopped. After pausing for blanking time as shown in step 2402, $Vel_{act}$ measurements are taken for a full time period as shown in step 2403. As in step 2404, the running average of the magnitude of the $Vel_{act}$ measurements is determined. In step 2405, the process considers whether or not the valve should open as determined by other elements of controller software. It should be noted that this determination could be made at other, or multiple points in the flow chart. If the system requires the valve to open, then the dislodgement detection process is terminated (and the valve may be opened). Otherwise, the controller compares the running average to the RMMT in step 2406. If the running average is greater than the RMMT, then it is determined that there is a high likelihood that the tube has been dislodged, and an alert may be activated as in step 2408 and the process terminates as in step 2409. If however, the running average is less than or equal to the RMMT, then another $Vel_{act}$ measurement is taken as in step 2407. The running average of the magnitude of Vex act measurements is updated by including the new $Vel_{act}$ measurement and dropping the oldest $Vel_{act}$ measurement as in step 2404 and the process continues.

Other embodiments of the invention will be apparent to those skilled in the art from consideration of the specification and practice of the invention disclosed herein. It is intended that the specification and examples be considered as exemplary only, with a true scope of the invention being indicated by the following claims.

**Claims**

1. A method of detecting tubing in a solenoid actuated pinch valve comprising:

   measuring operation of an armature (1303) of the pinch valve; the method being **characterized by**:
   providing calibration data pertaining to the solenoid pinch valve (1101-1111, 1201-1211);
   calculating an amount of work performed by the armature of the solenoid actuator (1304-1307);
   comparing the amount of work performed to a threshold value (1308); and
   determining a presence of tubing in the solenoid pinch valve based on a result of the comparison (1309-1312).

2. The method of claim 1, further including:

   comparing the amount of work performed to a plurality of additional threshold values; and
   estimating, based upon the result of the comparison to the plurality of threshold values, at least any one of a tubing hardness, a tubing type, or a pressure of fluid within the tubing.

3. The method of claim 1, wherein calculating the amount of work performed includes aggregating a plurality of incremental amounts of work performed (1307).

4. The method of claim 3, wherein at least one of the plurality of incremental amounts of work performed is calculated by multiplying an incremental change in position of the armature by an incremental amount of force exerted on the armature (1306).

5. The method of claim 4, wherein the incremental change in position is determined using an optical aperture sensor (206).

6. The method of claim 4, wherein the incremental amount of force is determined by multiplying a value indicative of a difference between a measured current value at which the armature does not accelerate (1304) and a null current value with a dF/di value indicative of the change in force of the armature relative to the change in current for a particular position of the armature (1305).

7. The method of claim 6, wherein:

   the null current value is dependent on a position of the armature, and
   the dF/di value is dependent on a position of the armature.

8.  The method of claim 7, wherein either the dF/di value or the null current value is further dependent on a direction in which the armature is moving.

9.  The method of claim 7, wherein either the dF/di value or the null current value is further dependent on an orientation of the solenoid actuator with respect to ground.

10. The method of claim 3, further including:

    controlling the armature as to reach and maintain a substantially steady armature velocity, wherein the aggregating a plurality of incremental amounts of work only occurs after the substantially steady armature velocity is reached.

11. The method of claim 4, wherein:

    the aggregating a plurality of incremental amounts of work performed occurs iteratively after each incremental amount of work is calculated, unless the presence of tubing has been determined; and
    the comparing the amount of work performed to threshold value is repeated after each iterative aggregation, unless the presence of tubing has been determined.

12. The method of claim 11, further including:

    placing the solenoid actuator into relax state if the presence of tubing has been determined.

13. A system for detecting tubing (102) in a solenoid actuated pinch valve, comprising:

    a solenoid actuator with an armature (208/210), wherein the solenoid actuator is configured to receive the tubing (102) and to measure operation of the armature; and
    a data storage device (205);
    **characterized in that** the data storage device contains calibration data pertainirig to the solenoid actuator; and
    wherein the system further comprises a controller configured to

        calculate an amount of work performed by the armature,
        compare the amount of work performed by the armature to a threshold value, and
        determine a presence of tubing in the solenoid pinch valve based on the comparison results.

14. The system as in claim 13, wherein the controller is further configured to calculate the amount of work performed by aggregating a plurality of incremental amounts of work performed.

15. The system as in claim 14, wherein the controller is further configured to calculate an incremental amount of work performed by multiplying an incremental change in position of the armature by an incremental amount of force exerted on the armature.

16. The system as in claim 15, wherein:

    the controller is further configured to calculate the incremental amount of force by multiplying a value indicative of the difference between a measured current value and a null current value at which the armature does not accelerate with a dF/di value indicative of the change in force of the armature relative to the change in current for a particular position of the armature.

17. The system as in claim 16, further comprising:

    an optical aperture sensor (206), wherein the optical aperture sensor is coupled to the solenoid actuator and the controller; wherein:

        the optical aperture sensor is configured to transmit to the controller a plurality of position data indicative of one or more positions of the armature, and
        the controller is further configured to determine the incremental change in position based upon the plurality of data received.

**18.** The system as in claim 17, wherein:

the controller is further configured to retrieve, from the data storage device, a null current value dependent on a position of the armature sensed by the optical aperture sensor; and
the controller is further configured to retrieve, from the data storage device, a dF/di value dependent on the position of the armature sensed by the optical aperture sensor.

**19.** The system as in claim 15, wherein the controller is further configured to:

iteratively aggregate the plurality of incremental amounts of work performed after each incremental amount of work is calculated, unless the presence of tubing has been determined; and
compare the amount of work performed to a threshold value after each iteration, unless the presence of tubing has been determined.

**20.** The system as in claim 19, wherein the controller is further configured to:

place the solenoid actuator into relax state if the presence of tubing has been determined.


**Patentansprüche**

**1.** Verfahren zur Erkennung einer Rohrleitung in einem Quetschventil, das durch einen Elektromagneten angetrieben wird, umfassend:

Messen des Betriebs eines Ankers (1303) des Quetschventils;
wobei das Verfahren **gekennzeichnet ist durch**:

Bereitstellen von Kalibrierungsdaten, die das Magnetquetschventil (1101-1111, 1201-1211) betreffen;
Berechnen einer **durch** den Anker des Magnetantriebs (1304-1307) ausgeführten Arbeitsmenge;
Vergleichen der ausgeführten Arbeitsmenge mit einem Schwellenwert (1308); und
Bestimmen der Präsenz einer Rohrleitung in dem Magnetquetschventil auf der Basis eines Ergebnisses des Vergleichs (1309-1312).

**2.** Verfahren nach Anspruch 1, ferner umfassend:

Vergleichen der ausgeführten Arbeitsmenge mit einer Mehrzahl weiterer Schwellenwerte; und
Schätzen auf der Basis des Ergebnisses des Vergleichs mit einer Mehrzahl von Schwellenwerten wenigstens eines Wertes einer Rohrleitungshärte, eines Rohrleitungstyps oder eines Drucks von Fluid in der Rohrleitung.

**3.** Verfahren nach Anspruch 1, wobei das Berechnen der ausgeführten Arbeitsmenge das Kumulieren einer Mehrzahl inkrementeller ausgeführter Arbeitsmengen (1307) aufweist.

**4.** Verfahren nach Anspruch 3, wobei wenigstens eine der Mehrzahl inkrementeller ausgeführter Arbeitsmengen berechnet wird durch Multiplizieren einer inkrementellen Positionsveränderung des Ankers mit einer inkrementellen, auf den Anker (1306) ausgeübten Kraftmenge.

**5.** Verfahren nach Anspruch 4, wobei die inkrementelle Veränderung der Position bestimmt wird unter Verwendung eines Apertursensors (206).

**6.** Verfahren nach Anspruch 4, wobei die inkrementelle Kraftmenge bestimmt wird durch Multiplizieren eines Wertes, der eine Differenz zwischen einem gemessenen Stromwert, bei dem sich der Anker nicht beschleunigt (1304) und einem Nullstromwert anzeigt, mit einem dF/di-Wert, der die Kraftveränderung des Ankers im Verhältnis zur Stromveränderung für eine bestimmte Position des Ankers (1305) anzeigt.

**7.** Verfahren nach Anspruch 6, wobei:

der Nullstromwert abhängig ist von einer Position des Ankers; und
der dF/di-Wert abhängig ist von einer Position des Ankers.

**8.** Verfahren nach Anspruch 7, wobei der dF/di-Wert oder der Nullstromwert ferner abhängig ist von einer Richtung, in die sich der Anker bewegt.

**9.** Verfahren nach Anspruch 7, wobei der dF/di-Wert oder der Nullstromwert ferner abhängig ist von einer Ausrichtung des Magnetantriebs im Verhältnis zum Boden.

**10.** Verfahren nach Anspruch 3, ferner umfassend:

Steuern des Ankers, so dass eine im Wesentlichen konstante Ankergeschwindigkeit erreicht und aufrechterhalten wird.

**11.** Verfahren nach Anspruch 4, wobei:

das Kumulieren einer Mehrzahl inkrementeller ausgeführter Arbeitsmengen iterativ nach der Berechnung jeder inkrementellen Arbeitsmenge eintritt, sofern nicht die Präsenz einer Rohrleitung bestimmt worden ist; und das Vergleichen der ausgeführten Arbeitsmenge mit einem Schwellenwert nach jeder iterativen Kumulation wiederholt wird, sofern nicht die Präsenz einer Rohrleitung bestimmt worden ist.

**12.** Verfahren nach Anspruch 11, ferner umfassend:

das Platzieren des Magnetantriebs in einem Ruhezustand, wenn die Präsenz einer Rohrleitung bestimmt worden ist.

**13.** System zur Erkennung einer Rohrleitung (102) in einem Quetschventil, das durch einen Elektromagneten angetrieben wird, umfassend:

einen Magnetantrieb mit einem Anker (208/210), wobei der Magnetantrieb so konfiguriert ist, dass er die Rohrleitung (102) aufnimmt und den Betrieb des Ankers misst; und eine Datenspeichervorrichtung (205); **dadurch gekennzeichnet, dass** die Datenspeichervorrichtung Kalibrierungsdaten aufwiest, die den Magnetantrieb betreffen; und wobei das System ferner ein Steuereinrichtung umfasst, die so konfiguriert ist, dass sie folgendes vornimmt:

Berechnen einer durch den Anker ausgeführten Arbeitsmenge; Vergleichen der durch den Anker ausgeführten Arbeitsmenge mit einem Schwellenwert; und Bestimmen der Präsenz einer Rohrleitung in dem Magnetquetschventil auf der Basis der Vergleichsergebnisse.

**14.** System nach Anspruch 13, wobei die Steuereinrichtung ferner so konfiguriert ist, dass sie die ausgeführte Arbeitsmenge berechnet, indem eine Mehrzahl inkrementeller ausgeführter Arbeitsmengen kumuliert wird.

**15.** System nach Anspruch 14, wobei die Steuereinrichtung ferner so konfiguriert ist, dass sie eine inkrementelle ausgeführte Arbeitsmenge berechnet durch Multiplizieren einer inkrementellen Veränderung der Position des Ankers mit einer auf den Anker ausgeübten inkrementellen Arbeitsmenge.

**16.** System nach Anspruch 15, wobei:

die Steuereinrichtung ferner so konfiguriert ist, dass sie die inkrementelle Kraftmenge berechnet, indem ein Wert, der die Differenz zwischen einem gemessenen Stromwert und einem Nullstromwert, bei dem sich der Anker nicht beschleunigt, mit einem dF/di-Wert multipliziert, der die Kraftveränderung des Ankers im Verhältnis zu der Veränderung des Stroms für eine bestimmte Position des Ankers anzeigt.

**17.** System nach Anspruch 16, ferner umfassend:

einen Apertursensor (206), wobei der Apertursensor mit dem Magnetantrieb und der Steuereinrichtung gekoppelt ist; wobei:

der Apertursensor so konfiguriert ist, dass er eine Mehrzahl von Positionsdaten, die eine oder mehrere Positionen des Ankers anzeigen, zu der Steuereinrichtung übermittelt; und wobei
die Steuereinrichtung ferner so konfiguriert ist, dass sie die inkrementelle Veränderung der Position auf der Basis der empfangenen Mehrzahl von Daten bestimmt.

**18.** System nach Anspruch 17, wobei:

die Steuereinrichtung ferner so konfiguriert ist, dass sie von der Datenspeichervorrichtung einen Nullstromwert abruft, der von einer Position des Ankers abhängig ist, die durch den Apertursensor gemessen wird; und wobei die Steuereinrichtung ferner so konfiguriert ist, dass sie von der Datenspeichervorrichtung einen dF/di-Wert abruft, der von der Position des Ankers abhängig ist, die durch den Apertursensor gemessen wird.

**19.** System nach Anspruch 15, wobei die Steuereinrichtung ferner so konfiguriert ist, dass sie folgendes vornimmt:

iteratives Kumulieren der Mehrzahl inkrementeller ausgeführter Arbeitsmengen nach der Berechnung jeder inkrementellen Arbeitsmenge, sofern nicht die Präsenz einer Rohrleitung bestimmt worden ist; und Vergleichen der ausgeführten Arbeitsmenge mit einem Schwellenwert nach jeder Iteration, sofern nicht die Präsenz einer Rohrleitung bestimmt worden ist.

**20.** System nach Anspruch 19, wobei die Steuereinrichtung ferner so konfiguriert ist, dass sie folgendes vornimmt:

Platzieren des Magnetantriebs in einem Ruhezustand, wenn die Präsenz einer Rohrleitung bestimmt worden ist.

**Revendications**

**1.** Procédé de détection de tube dans une électrovanne à manchon comprenant l'étape consistant à :

mesurer le fonctionnement d'une armature (1303) de la vanne à manchon ;
le procédé étant **caractérisé par** les étapes consistant à :

fournir des données d'étalonnage concernant l'électrovanne à manchon (1101-1111, 1201-1211) ;
calculer une quantité de travail effectué par l'armature de l'actionneur électromagnétique (1304-1307) ;
comparer la quantité de travail effectué à une valeur seuil (1308) ; et
déterminer une présence de tube dans l'électrovanne à manchon en fonction d'un résultat de la comparaison (1309-1312).

**2.** Procédé selon la revendication 1, comprenant en outre les étapes consistant à :

comparer la quantité de travail effectué à une pluralité de valeurs seuils supplémentaires ; et
estimer, en fonction du résultat de la comparaison à la pluralité de valeurs seuils, au moins l'un parmi la dureté de tube, le type de tube ou une pression de fluide dans le tube.

**3.** Procédé selon la revendication 1, le calcul de la quantité de travail effectué comprenant l'étape consistant à totaliser une pluralité de quantités incrémentielles de travail effectué (1307).

**4.** Procédé selon la revendication 3, au moins l'une de la pluralité de quantités incrémentielles de travail effectué étant calculée en multipliant une variation incrémentielle de la position de l'armature par une quantité incrémentielle de force exercée sur l'armature (1306).

**5.** Procédé selon la revendication 4, la variation incrémentielle de la position étant déterminée à l'aide d'un capteur d'ouverture optique (206).

**6.** Procédé selon la revendication 4, la quantité incrémentielle de force étant déterminée en multipliant une valeur indiquant une différence entre la valeur de courant mesurée à laquelle l'armature n'accélère pas (1304) et une valeur de courant nulle par une valeur dF/di indiquant la variation de la force de l'armature par rapport à la variation du courant pour une position particulière de l'armature (1305).

**7.** Procédé selon la revendication 6 :

la valeur de courant nulle dépendant de la position de l'armature, et
la valeur dF/di dépendant de la position de l'armature.

8. Procédé selon la revendication 7, la valeur dF/di ou la valeur de courant nulle dépendant en outre d'une direction dans laquelle se déplace l'armature.

9. Procédé selon la revendication 7, la valeur dF/di ou la valeur de courant nulle dépendant en outre d'une orientation de l'actionneur électromagnétique par rapport au sol.

10. Procédé selon la revendication 3, comprenant en outre l'étape consistant à :

   commander l'armature de sorte à atteindre et à maintenir une vitesse d'armature sensiblement constante, la totalisation d'une pluralité de quantités incrémentielles de travail ne se produisant qu'après que la vitesse d'armature sensiblement stable est atteinte.

11. Procédé selon la revendication 4 :

   la totalisation d'une pluralité de quantités incrémentielles de travail effectué se produisant de façon itérative après que chaque quantité incrémentielle de travail est calculée, à moins que la présence de tube n'ait été déterminée ; et
   la comparaison de la quantité de travail effectué à la valeur seuil étant répétée après chaque totalisation itérative, à moins que la présence de tube n'ait été déterminée.

12. Procédé selon la revendication 11, comprenant en outre l'étape consistant à :

   placer l'actionneur électromagnétique à l'état détendu si la présence de tube a été déterminée.

13. Système de détection de tube (102) dans une électrovanne à manchon, comprenant :

   un actionneur électromagnétique avec une armature (208/210), l'actionneur électromagnétique étant conçu pour recevoir le tube (102) et mesurer le fonctionnement de l'armature ; et
   un dispositif de stockage de données (205) ;
   **caractérisé en ce que** le dispositif de stockage de données contient des données d'étalonnage concernant l'actionneur électromagnétique ; et
   le système comprenant en outre un dispositif de commande conçu pour

   calculer une quantité de travail effectué par l'armature,
   comparer la quantité de travail effectué par l'armature à une valeur seuil, et
   déterminer une présence de tube dans l'électrovanne à manchon en fonction des résultats de la comparaison.

14. Système selon la revendication 13, le dispositif de commande étant en outre conçu pour calculer la quantité de travail effectué en totalisant une pluralité de quantités incrémentielles de travail effectué.

15. Système selon la revendication 14, le dispositif de commande étant en outre conçu pour calculer une quantité incrémentielle de travail effectué en multipliant une variation progressive de la position de l'armature par une quantité incrémentielle de force exercée sur l'armature.

16. Système selon la revendication 15 :

   le dispositif de commande étant en outre conçu pour calculer la quantité incrémentielle de force en multipliant une valeur indiquant une différence entre une valeur de courant mesurée et une valeur de courant nulle à laquelle l'armature n'accélère pas par une valeur dF/di indiquant la variation de la force de l'armature par rapport à la variation du courant pour une position particulière de l'armature.

17. Système selon la revendication 16, comprenant en outre :

   un capteur d'ouverture optique (206), le capteur d'ouverture optique étant couplé à l'actionneur électromagnétique et au dispositif de commande :

le capteur d'ouverture optique étant conçu pour transmettre au dispositif de commande une pluralité de données de position indiquant une ou plusieurs positions de l'armature, et
le dispositif de commande étant en outre conçu pour déterminer la variation progressive de la position en fonction de la pluralité de données reçues.

**18.** Système selon la revendication 17 :

le dispositif de commande étant en outre conçu pour récupérer, à partir du dispositif de stockage de données, une valeur de courant nulle dépendant d'une position de l'armature détectée par le capteur d'ouverture optique ; et
le dispositif de commande étant en outre conçu pour récupérer, du dispositif de stockage de données, une valeur dF/di dépendant de la position de l'armature détectée par le capteur d'ouverture optique.

**19.** Système selon la revendication 15, le dispositif de commande étant en outre conçu pour :

totaliser de façon itérative la pluralité de quantités incrémentielles de travail effectué après que chaque quantité incrémentielle de travail est calculée, à moins que la présence de tube n'ait été déterminée ; et
comparer la quantité de travail effectué à une valeur seuil après chaque itération, à moins que la présence de tube n'ait été déterminée.

**20.** Système selon la revendication 19, le dispositif de commande étant en outre conçu pour :

placer l'actionneur électromagnétique à l'état détendu si la présence de tube a été déterminée.

Fig. 1

Fig. 2

# Fig. 3

| MODEL NO. | 2400-H-AB-V107 | Test Completed by: | |
|---|---|---|---|
| ARMATURE MASS(LB) | 0.377 | | |
| EEPROM S/N | 4100000026B2C543 | Preformed on: | 2/25/2011 |
| SWITCH S/N | FD00000005405F3A | Valve built on: | 2/17/2011 |
| RDAC | 38 | Customer: | |
| STROKE LENGTH (inches) | 0.2400 | Serial Number: | 41685-10225-001 |
| TEMP (deg F) | 69.7 | | |
| RESISTANCE (Ohms) | 3.418 | | |

# Fig. 4

| TRIAL | PULL-IN CURRENT (A) | HOLD-IN POSITION (IN) | HOLD-IN (.001 DEV) CURRENT (A) | DROP-OUT CURRENT (A) | DROP-OUT POSITION (IN) |
|-------|---------------------|-----------------------|-------------------------------|----------------------|------------------------|
| 1 | 4.313 | 0.2400 | 1.319 | 0.810 | 0.2310 |
| 2 | 4.313 | 0.2400 | 1.319 | 0.773 | 0.2305 |
| 3 | 4.318 | 0.2400 | 1.391 | 0.815 | 0.2305 |
| MEAN | 4.315 | 0.2400 | 1.343 | 0.799 | 0.2307 |

# Fig. 5

| SENSOR SWEEP DATA | |
|:---:|:---:|
| POSITION (IN) | SENSOR-OUTPUT (V) |
| -0.0005 | 2.7040 |
| 0.0045 | 2.6810 |
| 0.0125 | 2.5670 |
| 0.0215 | 2.4260 |
| 0.0305 | 2.2780 |
| 0.0400 | 2.1150 |
| 0.0485 | 1.9660 |
| 0.0575 | 1.8277 |
| 0.0665 | 1.6894 |
| 0.0750 | 1.5562 |
| 0.0845 | 1.4261 |
| 0.0935 | 1.2989 |
| 0.1025 | 1.1722 |
| 0.1115 | 1.0456 |
| 0.1205 | 0.9334 |
| 0.1290 | 0.8299 |
| 0.1380 | 0.7297 |
| 0.1475 | 0.6377 |
| 0.1565 | 0.5504 |
| 0.1655 | 0.4703 |
| 0.1740 | 0.3939 |
| 0.1830 | 0.3275 |
| 0.1920 | 0.2686 |
| 0.2010 | 0.2158 |
| 0.2105 | 0.1751 |
| 0.2190 | 0.1428 |
| 0.2280 | 0.1150 |
| 0.2360 | 0.0943 |

## Fig. 6

| Data Point | | "y" Measurements | | | "z" Measurements | | | Derived Values | | |
|---|---|---|---|---|---|---|---|---|---|---|
| | | POSITION (IN) | FORCE (LBF) | CURRENT (A) | POSITION (IN) | FORCE (LBF) | CURRENT (A) | NULL POSITION (IN) | NULL CURRENT (A) | di/dF (A/LBF) |
| 1 | Trial 1 | 0.0205 | 2.18 | 3.586 | 0.023 | -1.9 | 4.796 | 0.0218 | 4.2325 | 0.296569 |
| 2 | Forward | 0.073 | 2.16 | 3.322 | 0.075 | -1.96 | 4.283 | 0.0740 | 3.8258 | 0.233252 |
| 3 | Pass | 0.123 | 2.04 | 2.888 | 0.125 | -1.90 | 3.585 | 0.1240 | 3.2489 | 0.176904 |
| 4 | +1 -> -1 | 0.173 | 2.06 | 2.195 | 0.175 | -1.93 | 2.612 | 0.1738 | 2.4103 | 0.104511 |
| 5 | | 0.212 | 2.18 | 1.446 | 0.215 | -1.99 | 1.607 | 0.2136 | 1.5302 | 0.038777 |
| 6 | Trial 1 | 0.025 | -2.18 | 4.395 | 0.0245 | 1.9 | 3.124 | 0.0247 | 3.7159 | 0.311520 |
| 7 | Backward | 0.0775 | -2.15 | 3.871 | 0.0765 | 1.9 | 2.862 | 0.0770 | 3.3354 | 0.249136 |
| 8 | Pass | 0.128 | -2.16 | 3.187 | 0.127 | 1.93 | 2.424 | 0.1272 | 2.7840 | 0.186553 |
| 9 | -1 -> +1 | 0.178 | -2.19 | 2.265 | 0.177 | 1.93 | 1.783 | 0.1770 | 2.0090 | 0.116893 |
| 10 | | 0.218 | -2.19 | 1.272 | 0.216 | 1.95 | 1.067 | 0.2167 | 1.1633 | 0.049493 |
| | | | | | | | Pull in | 0.0000 | 4.3147 | 0.323048 |
| | | | | | | | Drop out | 0.2307 | 0.7993 | 0.049493 |
| | | | | | | | Pull out | 0.0000 | 3.8961 | 0.341057 |
| | | | | | | | Hold in | 0.2400 | 1.3430 | 0.038777 |

EP 2 518 575 B1

# Fig. 7

## di/dF as a function of position

di/dF(x)[Forward] = -0.053604x² - 1.171597x + 0.323048     di/dF(x)[Backwards] = -0.480439x² - 1.153186x + 0.341057

□Forward
△Backwards

## Fig. 8

*Null current as a function of position*

EP 2 518 575 B1

If-Null(x) = -32.677071x² - 4.539447x + 4.314667          Ib-Null(x) = -45.299299x² - 2.976078x + 3.896060

□Forward
△Backwards

Position(in)

# Fig. 9

### Sensor output as a function of position

$$y = -91194.346105x^6 + 70346.663203x^5 - 21118.852406x^4 + 3170.706922x^3 - 218.569136x^2 - 9.989095x + 2.713961$$

$$R^2 = 0.999939$$

Position(in)

EP 2 518 575 B1

# Fig. 10

## Position as a function of sensor output

$y = 0.006608x^6 - 0.066377x^5 + 0.261544x^4 - 0.517190x^3 + 0.551484x^2 - 0.384958x + 0.265212$

$R^2 = 0.999746$

Output(V)

EP 2 518 575 B1

# Fig. 11

1101 — ATTACH SOLENOID PINCH VALVE TO CALIBRATION EQUIPMENT (IF NOT ATTACHED)

1102 — POSITION ARMATURE SUCH THAT VALUE IS MOSTLY CLOSED

1103 — INCREASE CURRENT UNTIL ARMATURE EXERTS APPROXIMATELY 2 POUNDS OF FORCE (POSITIVE FORCE TENDS TO CLOSE THE VALVE)

1104 — MEASURE CURRENT
MEASURE ARMATURE POSITION
MEASURE FORCE

1105 — INCREASE CURRENT UNTIL ARMATURE EXERTS APPROXIMATELY -2 POUNDS OF FORCE (NEGATIVE FORCE TENDS TO OPEN THE VALVE)

1106 — MEASURE CURRENT
MEASURE ARMATURE POSITION
MEASURE FORCE

1107 — IS ANOTHER DATA POINT DESIRED IN THIS PASS?

NO

YES

1108 — CAN THE VALVE BE OPENED FURTHER?

NO

1111 — TRIAL COMPLETED

1109 — YES

SET CURRENT TO 0

1110 — REPOSITION ARMATURE SO THAT VALVE IS SLIGHTLY MORE OPEN

# Fig. 12

1201 — ATTACH SOLENOID PINCH VALVE TO CALIBRATION EQUIPMENT (IF NOT ATTACHED)

1202 — POSITION ARMATURE SUCH THAT VALUE IS MOSTLY CLOSED

1203 — INCREASE CURRENT UNTIL ARMATURE EXERTS FORCE OF LESS THAN -4 POUNDS (NEGATIVE FORCE TENDS TO OPEN THE VALVE)

1204 — DECREASE CURRENT UNTIL ARMATURE EXERTS APPROXIMATELY -2 POUNDS OF FORCE

1205 — MEASURE CURRENT
MEASURE ARMATURE POSITION
MEASURE FORCE

1206 — DECREASE CURRENT UNTIL ARMATURE EXERTS APPROXIMATELY 2 POUNDS OF FORCE (POSITIVE FORCE TENDS TO CLOSE THE VALVE)

1207 — MEASURE CURRENT
MEASURE ARMATURE POSITION
MEASURE FORCE

1208 — IS ANOTHER DATA POINT DESIRED IN THIS PASS?
— NO
— YES

1209 — CAN THE VALVE BE OPENED FURTHER?
— NO
— YES

1212 — TRIAL COMPLETED

1210 — SET CURRENT TO 0

1211 — REPOSITION ARMATURE SO THAT VALVE IS SLIGHTLY MORE OPEN

# Fig. 13

1301 — SET TOTAL WORK TO ZERO

1302 — VALVE IS CLOSING

1303 — MEASURE $I_{actual}$
MEASURE ARMATURE POSITION, X

1304 — SUBTRACT $I_{null}(X)$ FROM ACTUAL CURRENT
TO YIELD DEVIATION CURRENT

1305 — MULTIPLY DEVIATION CURRENT BY
$dF/di_B(X)$ TO YIELD INCREMENTAL AMOUNT
OF FORCE

1306 — MULTIPLY INCREMENTAL AMOUNT OF FORCE
BY CHANGE IN POSITION SINCE THE
PREVIOUS CYCLE TO YIELD INCREMENTAL
AMOUNT OF WORK

1307 — ADD NEWLY CALCULATED INCREMENTAL
AMOUNT OF WORK TO TOTAL WORK

1308 — IS TOTAL
WORK GREATER THAN
THE THRESHOLD WORK
VALUE?

YES

1309 — TUBING
DETECTED

NO

1310 — IS THE
SOLENOID PINCH
VALVE FULLY
CLOSED?

YES

1311 — NO TUBING IN
SLOT

1312 — CLOSING PRESUMED TO CONTINUE

NO

## Fig. 14

Armature position, current, net force and work as a function of time
control law=velocity.hold, direction=close, orientation=horizontal, tubing=no

F(lbf)
i(A)
W(oz-in)
x(in)

armature position (in)

work (oz-in), force (lbf), current (A)

time (seconds)

## Fig. 15

Armature position, current, net force and work as a function of time
control law=velocity.hold, direction=close, orientation=horizontal, tubing=yes

## Fig. 16

Armature position, current, net force and work as a function of time
control law=velocity.hold, direction=close, orientation=vertical, tubing=no

EP 2 518 575 B1

Fig. 17

Armature position, current, net force and work as a function of time
control law=velocity.hold, direction=close, orientation=vertical, tubing=yes

# Fig. 18

Armature position and current as a function of time
control law=velocity.hold, direction=open, orientation=horizontal, tubing=no

- - i(A)

------ x(in)

EP 2 518 575 B1

## Fig. 19

Armature position and current as a function of time
control law=velocity.hold, direction=open, orientation=horizontal, tubing=yes

-- -- i(A)
------ x(in)

time (seconds)

Fig. 20

Fig. 21

Armature position and current as a function of time
control law=velocity.hold, direction=open, orientation=vertical, tubing=yes

- - i(A)
------ x(in)

EP 2 518 575 B1

# Fig. 22

| Raw Data Output from Energy Measurement Function in Valve Control Kernel | | | | | |
|---|---|---|---|---|---|
| Trial | No Tubing Loaded | Silicone Tubing | PharMed Tubing | PVC | Worn P&R Tubing w/H2O |
| 1 | -31,637 | 6,654 | 5,349 | -8,733 | -11,366 |
| 2 | -37,377 | 2,110 | -1,793 | -6,448 | -11,510 |
| 3 | -36,376 | -432 | -4,494 | -5,885 | -12,770 |
| 4 | -34,236 | -53 | -4,879 | -6,450 | -15,662 |
| 5 | -34,064 | -1,729 | -7,708 | -5,237 | -12,833 |
| 6 | -33,231 | -3,734 | -7,573 | -8,932 | -13,677 |
| 7 | -36,119 | -4,722 | -4,946 | -4,951 | -15,710 |
| 8 | -33,942 | -3,679 | -8,893 | -13,498 | -12,749 |
| 9 | -31,802 | -5,611 | -9,087 | -10,351 | -12,995 |
| 10 | -35,457 | -5,661 | -4,712 | -13,093 | -13,606 |
| Average | -34,424 | -1,686 | -4,874 | -8,358 | -13,288 |
| Minimum | -37,377 | -5,661 | -9,087 | -13,498 | -15,710 |
| Maximum | -31,637 | 6,654 | 5,349 | -4,951 | -11,366 |
| Std. Dev. | 1,913 | 3,899 | 4,261 | 3,128 | 1,471 |

| Calibration and Unit Adjusted Data (Energy units are pounds of force x 0.001") | | | | | |
|---|---|---|---|---|---|
| Trial | No Tubing Loaded | Silicone Tubing | PharMed Tubing | PVC | Worn P&R Tubing w/H2O |
| 1 | 2.658 | 39.175 | 37.931 | 24.501 | 21.990 |
| 2 | -2.816 | 34.842 | 31.119 | 26.680 | 21.853 |
| 3 | -1.861 | 32.417 | 28.544 | 27.217 | 20.651 |
| 4 | 0.179 | 32.779 | 28.176 | 26.678 | 17.893 |
| 5 | 0.343 | 31.180 | 25.478 | 27.835 | 20.591 |
| 6 | 1.138 | 29.268 | 25.607 | 24.311 | 19.786 |
| 7 | -1.616 | 28.326 | 28.113 | 28.108 | 17.847 |
| 8 | 0.460 | 29.321 | 24.348 | 19.957 | 20.671 |
| 9 | 2.501 | 27.478 | 24.163 | 22.958 | 20.436 |
| 10 | -0.985 | 27.431 | 28.336 | 20.343 | 19.854 |
| Average | 0.000 | 31.222 | 28.182 | 24.859 | 20.157 |
| Minimum | -2.816 | 27.431 | 24.163 | 19.957 | 17.847 |
| Maximum | 2.658 | 39.175 | 37.931 | 28.108 | 21.990 |
| Std. Dev. | 1.824 | 3.718 | 4.063 | 2.983 | 1.402 |
| Average-3σ | -5.473 | 20.068 | 15.991 | 15.911 | 15.950 |
| Average+3σ | 5.473 | 42.376 | 40.372 | 33.807 | 24.364 |

# Fig. 23

2301 — MEASURE POSITION

2302 — DETERMINE VELOCITY

2303 — DETERMINE VELOCITY ERROR

2304 — DETERMINE DESIRED CHANGE IN FORCE

2305 — DETERMINE CURRENT NEEDED TO PROVIDE DESIRED CHANGE IN FORCE

2306 — DETERMINE OUTPUT VOLTAGE NECESSARY TO PROVIDE DESIRED CURRENT

2307 — CONTROLLER PROVIDES CALCULATED OUTPUT VOLTAGE TO SOLENOID PINCH VALVE

2308 — CLOSING, OR IN SOME CASES OPENING, PRESUMED TO CONTINUE

# Fig. 24

VALVE CLOSED
WITH TUBING — 2401

WAIT FOR
BLANKING TIME — 2402

Vel$_{act}$ MEASUREMENT
TAKEN FOR FULL TIME
PERIOD — 2403

NEW Vel$_{act}$
MEASUREMENT TAKEN — 2407

DETERMINE RUNNING
AVERAGE OF THE
MAGNITUDE OF Vel$_{act}$
MEASUREMENTS — 2404

IS VALUE
DIRECTED TO
OPEN? — 2405

NO

IS THE
RUNNING
AVERAGE
GREATER
THAN RMMT? — 2406

NO

YES

2409

DETECTION
PROCESS
TERMINATED

ALERT — 2408

YES

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2010020074 A1
- US 20100188667 A1
- WO 03070314 A
- US 5977732 A

**Non-patent literature cited in the description**

- Practical Pinch Detection Algorithm for Low-Cost Anti-Pinch Window Control System. **HYE-JIN LEE et al.** INDUSTRIAL TECHNOLOGY, 2005. ICIT 2005. IEEE INTERNATIONAL CONFERENCE ON HONG KONG. IEEE, 14 December 2005, 995-1000